# EUROPÄISCHE PATENTANMELDUNG

(11) **EP 4 736 821 A1**
(43) Veröffentlichungstag der Anmeldung: **06.05.2026**
(21) Anmeldenummer: 25211835.1
(22) Anmeldetag: 28.10.2025
(51) Int. Cl.: A61F 5/00

(54) **SYSTEM FÜR EINE ENDOSKOPISCH UNTERSTÜTZTE, TRANS-ÖSOPHAGEALE APPLIKATION EINER DAUERHAFT IM KÖRPER VERBLEIBENDEN VORRICHTUNG FÜR DIE TRANS-DUODENALE, BYPASS-ARTIGE DURCHLEITUNG VON MAGENINHALT**

(30) Priorität: 29.10.2024 DE 102024003557
(71) Anmelder: Trans-Duodenal Concepts GmbH, 68199 Mannheim (DE)
(72) Erfinder: Kähler, Georg, 68199 Mannheim (DE)
(74) Vertreter: Küchler, Stefan

(57) **Zusammenfassung**

Die Erfindung richtet sich auf ein System für eine bypass-artige, trans-duodenale Durchleitung von Mageninhalt in die höheren Anteile des Jejunums (JE), umfassend
a) eine im Körper des Patienten verbleibende, trans-duodenale Bypass-Einheit (BE), umfassend (i) eine trans-duodenale Baugruppe (TDG) mit einer Schlauchfolie (7) für die Durchleitung von Mageninhalt in das Jejunum (JE), sowie (ii) für eine Fixierung oder Verankerung der Bypass-Einheit (BE) im Bereich des Schließmuskels des Magenausgangs (Pylorus) eine trans-pylorische Baugruppe (TPG) mit einer schlauch- oder rohrartigen Schaftkomponente (2), und
b) für die Applikation der trans-duodenalen Bypass-Einheit (BE), also für deren trans-ösophageale Passage bzw. Einbringung, deren trans-pylorische Positionierung, sowie deren trans-duodenale Entfaltung einer die Bypass-Funktion gewährleistenden Schlauchfolie eine trans-ösophageale Positionier-Einheit (PE) mit einer vorzugsweise schlauchförmigen Schaftkomponente (9), deren Länge derart bemessen ist, dass sie von extrakorporal durch den Ösophagus, den Magen und wenigstens bis zum Treitzschen Band im Bereich des aboralen Endes des Duodenums reicht, und deren Außendurchmesser zumindest in einem distalen Bereich kleiner ist als der Innendurchmesser der Schaftkomponente (2) der trans-pylorischen Baugruppe (TPG) der Bypass-Einheit (BE), sowie
c) einer von extra-korporal steuerbare Koppel-Einrichtung (KE), welche die trans-duodenale Bypass-Einheit (BE), insbesondere deren Schaftkomponente (2), auf der trans-ösophagealen Positionier-Einheit (PE), insbesondere auf deren Schaftkomponente (9), wahlweise koppelnd fixiert oder entkoppelnd freigibt.
sowie auf ein Verfahren unter Verwendung dieses Systems, wobei die trans-duodenale Schlauchfolie (7) der Bypass-Einheit (BE) durch die in aboraler Richtung vorgeschobene Positionier-Einheit (PE) in eine aboral des Treitzschen Bandes gelegene Position gebracht wird.

## Beschreibung

Die Erfindung beschreibt ein System für eine bypass-artige, trans-duodenale Durchleitung von Mageninhalt in die höheren Anteile des Jejunums, mit
a) einer im Körper verweilenden, trans-duodenalen Bypass-Einheit, umfassend (i) eine trans-pylorische Baugruppe für die Fixierung der Einheit im Bereich des Schließmuskels des Magenausgangs (Pylorus), sowie (ii) eine trans-duodenale Baugruppe für die Durchleitung von Mageninhalt in das Jejunum, sowie
b) einer Positionierungs-Einheit für die Applikation der trans-duodenalen Bypass-Einheit, also für deren trans-ösophageale Passage bzw. Einbringung, deren trans-pylorische Positionierung, sowie deren trans-duodenale Entfaltung einer die Bypass-Funktion gewährleistenden Schlauchfolie.

Die Adipositas (Fettleibigkeit) und vor allem ihre Folgeerkrankungen wie Diabetes mellitus Typ 2 und Leberverfettung, stellen weltweit ein zunehmendes medizinisches und sozialökonomisches Problem dar. Allein die Zahl der Diabetiker wird momentan weltweit mit ca. 600 Mio angegeben, von denen die große Mehrzahl kausal auf eine Adipositas zurückzuführen ist. Trotz weltweiter und umfassender Anstrengungen zur Vorbeugung und Behandlung nimmt die Zahl übergewichtiger Menschen seit Jahrzenten zu.

Für die medizinische Behandlung erschwerend ist, dass die Ursachen für die Adipositas und ihre pandemische Ausbreitung überwiegend unerforscht sind. Die genetische Disposition für Adipositas gilt als gesichert. Die Rolle des den Darm eines Patienten besiedelnden Mikrobioms bei der Entstehung von Übergewicht ist noch nicht wirklich verstanden.

Die bisherigen therapeutischen Möglichkeiten zur Behandlung einer Adipositas basieren auf:
- diätetischen und physischen Maßnahmen: Hier kommt in Betracht eine Verringerung der Energiebilanz durch verringerte Energieaufnahme einerseits und vermehrtem Energieverbrauch durch Sport und Bewegungsprogramm andererseits; der Erfolg dieses Konzeptes ist gering;
- medikamentöser Behandlung: Während Pankreas-Enzymblocker wegen ihrer Nebenwirkungen keine breite Anwendung gefunden haben, stehen derzeit neuentwickelte Insulinanaloga wie z.B. Semaglutid im Fokus des Interesses. In den Zulassungsstudien konnten zuverlässig Gewichtsverluste bis 35 % erzielt werden, nachhaltige Effekte waren jedoch nicht darstellbar. Die Medikamente müssten lebenslang eingenommen werden und über die Nebenwirkungen ist noch wenig bekannt;
- chirurgischen Verfahren: Diese basieren entweder auf einer Verkleinerung des Magens (Restriktion) oder auf einer Verkürzung des resorptiven Dünndarmabschnittes (Malabsorption), wobei immer das Duodenum umgangen wird (duodenaler Bypass). Diese Verfahren sind invasiv und irreversibel. Da das Ausmaß ihrer Wirksamkeit im Einzelfall nicht vorhergesagt werden kann, benötigen viele Patienten lebenslange Nachbetreuung. Für die Adipositas Grad III (Adipositas permagna mit einem BMI ≥ 40) haben die operativen Verfahren jedoch eine hohe Wirksamkeit sowohl hinsichtlich des Gewichtsverlustes und der Verbesserung der Lebensqualität als auch des Langzeitüberlebens und der Vermeidung von Folgeerkrankungen, einschließlich Diabetes und maligner Tumoren, unter Beweis gestellt.

In den vergangenen Jahren haben sich für die Therapie einer Adipositas zunehmend endoskopisch basierte, therapeutische Techniken etabliert, die es ermöglichen, die Effektivität chirurgischer Verfahren mit der geringen Invasivität flexibelendoskopischer Eingriffe, bei denen über natürliche Körperöffnungen in den Magendarmtrakt eingegangen wird, zu verbinden.

Das historisch älteste endoskopisch basierte Verfahren ist die Implantation eines Magenballons mit ca. 450-750 ccm Volumen, der eine anhaltende Magenfüllung gewährleisten und somit bei der Nahrungsaufnahme ein vorzeitiges Sättigungsgefühl erzeugen soll. Daten über einen langfristigen Behandlungserfolg gibt es zu dieser Methode nicht.

In den vergangenen drei bis fünf Jahren wurden zudem verschiedene Verfahren entwickelt, um das Magenvolumen durch endoskopisch gelegte Nähte zu verkleinern. Aufgrund früherer Studien zum Vergleich von laparoskopischen (d.h. durch eine Bauchhöhlenspiegelung durchgeführte) Magenfaltungen und Magenteilentfernungen wurde die Überlegenheit letzterer festgestellt. Insofern würde sich, selbst unter der Annahme, dass die endoskopischen Nähte eine dauerhafte Haltbarkeit hätten, die endoskopische Raffung des Magens nicht empfehlen.

Die endoskopische Schaffung einer Bypass-Situation ist naturgemäß schwieriger und deshalb noch nicht klinisch etabliert. Erste Ansätze bestehen in verschiedenen Verfahren zur thermischen Ablation der Duodenalschleimhaut, um auf diese Weise die Resorption von Kohlenhydraten als Trigger für die Insulinausschüttung auszuschalten.

Dem chirurgischen Prinzip der Bypass-Operation am nächsten kommen Verfahren zur intra-luminalen Überbrückung des Duodenums, die einen trans-duodenalen Bypass der Nahrung durch einen im Körper verweilenden, nicht-permeablen Folienschlauch ermöglichen. Hierfür hat sich der produktneutrale Begriff des "gastrointestinalen Liners" etabliert, der auch in der internationalen Klassifikation der Prozeduren in der Medizin (OPS) verwendet wird.

Das erste, bis 2018 kommerziell verfügbare Implantat dieser Art war das Produkt "Endo-Barrier" (jetzt "Reset") der Firma Gl Dynamics (jetzt "Morphic medical"). Abgesehen von der problematischen Art der Fixierung dieses Implantates im Duodenum durch scharfkantige, in die Duodenalschleimhaut eindringende Metallhaken, bestand eines der grundsätzlichen Anwendungsprobleme in der Ausrollung bzw. der vollständigen axialen Entfaltung des den Bypass gewährleistenden Folienschlauches in das Duodenum. Für die trans-duodenale Anlage der Schlauchfolie wurde eine röntgen-opake Kugel genutzt, deren Passage bis ins Jejunum jedoch sehr zeitaufwändig in der Anwendung war und eine Durchleuchtung mit einer entsprechenden Strahlenexposition erforderte. Wegen der enormen Schwierigkeit dieses Manövers hat der Hersteller zudem verlangt, dass alle Anwender zunächst mehrere Prozeduren im Tierversuch absolvieren, was heute tierschutzrechtlich nicht mehr vertretbar ist.

Die bisherige Historie endoskopisch applizierter, gastrointestinaler Liner-Vorrichtungen zeigt in der klinischen Anwendung die nachfolgenden Leistungsmerkmale als kritisch bzw. für den Anwendungserfolg wesentlich:
- Die Applikation der Vorrichtungen muss auch von ungeübten Anwendern rasch und sicher durchgeführt werden können;
- die Applikation sollte ohne radiologische Führung bzw. Kontrolle erfolgen;
- die Vorrichtung muss in einer möglichst atraumatischen und möglichst wenig irritierendenden Weise über Zeiträume von mehreren Monaten im Patienten verweilen können;
- die Fixierung der Vorrichtung im Übergangsbereich vom Magen zum Duodenum muss zuverlässig sein bzw. Dislokationen der Vorrichtung sicher ausschließen;
- die axiale Entfaltung des vorzugsweise folien-artig dünnwandigen Bypass-Schlauches über die gesamte Länge des Duodenums muss möglichst einfach, schnell und komplikationslos erfolgen.

Die vollständige axiale, trans-duodenale Entfaltung der den Bypass von Nahrung ermöglichenden Schlauchfolie stellt insbesondere für Anwender mit wenig endoskopischer Erfahrung eine schwierige, zeitaufwendige und unter Umständen letztlich nicht zu meisternde Herausforderung dar.

Eine vereinfachte trans-duodenale Entfaltung der Bypass-Schlauchkomponente gelingt im Rahmen eines gattungsgemäßen Systems durch die kombinierte Anwendung einer im Patienten verbleibenden Bypass-Einheit mit einer baulich und funktionell kompatiblen, katheterartigen Positionierungs-Einheit, wobei die zu applizierende Bypass-Einheit auf der Positionierungs-Einheit durch einen extrakorporal bedienbaren Mechanismus koppelnd fixiert oder entkoppelnd freigegeben werden kann.

Die vorliegende Erfindung beschreibt ein ballon-basiertes, trans-pylorisches Fixierungssystem, dass die im Patienten verweilende Bypass-Einheit über den Schließmuskel des Magens hinweg dislokationssicher verankert. Die entsprechende trans-pylorische Baugruppe der Einheit weist einen duodenalen, unmittelbar post-pylorisch positionierten Ballon auf. In der magenseitigen, prä-pylorischen Position, stehen dem duodenalen Ballon zwei konzentrisch angeordnete Ballons gegenüber. Während der duodenale Ballon eine Dislokation der Baugruppe in den Magen verhindert, schließen die beiden magenseitigen Ballons ein Abgleiten bzw. eine Dislokation der Bypass-Vorrichtung in das Duodenum aus.

Die trans-duodenale Baugruppe der Bypass-Einheit besteht im Wesentlichen aus einem ca. 60 bis 70 cm langen, besonders dünnwandig hergestellten Folienschlauch mit einem durchgängig zylindrischen Durchmesser von ca. 20 bis 30 mm, wie er in dieser Bauweise und Dimensionierung für vergleichbare trans-duodenale Bypass-Vorrichtungen typisch ist. Die Ausführung der Folienschlauchkomponente in der beschriebenen Länge gestattet eine über die Flexura duodenojejunalis (Treitzsches Band) hinausreichende Positionierung des distalen Folienschlauchendes und sichert so die Durchleitung bzw. Abgabe des Mageninhaltes in die oberen Anteile des Jejunums. Nach allgemeiner klinischer Erfahrung ist mit der Überwindung des Treitzschen Bandes nicht mehr damit zu rechnen, dass Darminhalt zum postpylorischen Duodenum oder zum Magen zurückfließt. Die propulsive Peristaltik reicht dann aus, um einen gerichteten Transport des Mageninhaltes in die tieferen Dünndarmanteile zu gewährleisten.

Die von extrakorporal geführte bzw. gesteuerte, vollständige trans-duodenale axiale Entfaltung der Schlauchkomponente wird insbesondere durch die membranartige Dünnwandigkeit der Schlauchfolie erschwert.

Etablierte Instrumente, wie z.B. ein flexibles Endoskop (Gastroskop, Enteroskop) können, bei entsprechender Übung des Anwenders, den anatomischen Bereich um das Treitzsche Band zwar mit ihrer Spitze erreichen und somit auch die Schlauchfolie jenseits des Treitzschen Bandes transportieren, entwickeln bei der Applikation aber derart hohe Reibungswiderstände, dass es beim Zurückziehen des Endoskopschaftes aus einer axial entfalteten Folienschlauchkomponente in der Regel zur Mitnahme der Schlauchfolie kommt bzw. diese dem Endoskopschaft anhaftet und ihre trans-duodenal entfaltete Lage wieder verliert. Die direkte, endoskopisch geführte axiale Entfaltung des Bypass-Schlauches ist für den erfolgreichen Einsatz in der klinischen Routine daher nur sehr eingeschränkt geeignet.

Techniken, die auf eine endoskopisch basierte oder sonstig schaftbasierte, trans-duodenale Einbringung der Bypass-Einheit verzichten und lediglich eine draht-basierte Komponente für den Vorschub der Bypass-Schlauchkomponente durch das Duodenum verwenden, sind im Stand der Technik beschrieben, haben allerdings den Nachteil einer geringen Schubsteifigkeit und sind daher von extrakorporal schlecht steuerbar.

Die vorliegende Erfindung beschreibt eine vereinfachte Bauweise einer Bypass-Vorrichtung, wie diese in ähnlicher Weise bereits in der WO 2016/067087 A2 offenbart ist. Dort wird eine für die dynamisch adaptiv dichtende und atraumatisch organverträgliche, trans-pylorische Fixierung vorteilhafte Bau- und Funktionsweise vorgestellt, wobei die Bypass-Vorrichtung mindestens eine Ballonkomponente aufweist, die derart auf einer trans-pylorisch durchleitenden Schaftkomponente aufgebracht ist, dass die Ballonkomponente bei der Befüllung bzw. Beaufschlagung mit Fülldruck eine zum Pylorus hin gerichtete Rollbewegung vollzieht, wobei gleichzeitig die radiale Expansion, insbesondere des duodenalseitigen Ballonkorpus, zu den anliegenden Geweben so weit wie möglich begrenzt werden kann, so dass die Wahrscheinlichkeit druckbedingter Schädigungen der jeweiligen Organstrukturen vermindert ist.

Im Rahmen der WO 2016/067087 A2 werden besondere Ausführungsformen der "axial gegenrollenden Bauweise" beschrieben, bei denen ein trans-pylorisches, schaft-artiges Durchleitungselement mit sowohl duodenal als auch gastrisch angeordneten Ballons ausgestattet ist. Die Offenbarung stellt unter anderem Ballonanordnungen vor, bei denen zwei Ballons in konzentrischer Weise angeordnet sind bzw. bei denen ein innenliegender Ballon vollständig von einem außen liegenden Ballon eingeschlossen ist. Die konzentrisch angeordneten Ballons sind jeweils separat befüllbar. Ferner werden optional in den Ballons deponierte Indikatorfarbstoffe beschrieben, die bei ihrer Freisetzung über den Darm aufgenommen und auf dem renalen Weg, für den Anwender als Farbveränderung im Urin erkennbar, ausgeschieden werden.

Ähnliche Bauformen mit mehreren, auf einem zentralen, durchleitenden Schaftelement aufgebrachten Ballons werden in der WO 2005/120363 A1 beschrieben. In der dortigen Fig. 21A wird eine konzentrische Ballonanordnung gezeigt. Hierbei hat lediglich der innere der beiden Ballons eine positionssichernde, haltende Funktion, während der äußere Ballon baulich für die Aufnahme und Freigabe von pharmakologisch wirksamen Substanzen konzipiert ist. Die Hülle des äußeren Ballons ist durchlässig und für den dauerhaften Einschluss von Medien nicht geeignet. Anders als in WO 2016/067087 A2 wird in der WO 2005/120363 A1 auf den Aspekt der Positionssicherung durch einen redundanten, sichernden zweiten Ballon nicht eingegangen.

Die WO 2016/067087 A2 beschreibt eine besondere Form der endoskopischen Platzierung einer ballonbasierten, trans-pylorisch fixierten, trans-duodenalen Bypass-Vorrichtung, Hierbei wird die gesamte zu applizierende Bypass-Vorrichtung auf der Oberfläche des distalen Endoskopschaftes aufgenommen, wobei der Endoskopschaft in das zentrale Lumen der Bypass-Vorrichtung eingesteckt wird und die Fixierung durch eine von extrakorporal befüllbare Kopplungsballon-Komponente zwischen dem Endoskopschaft und der Wandung des durchleitenden Lumens der Bypass-Einheit hergestellt wird. Der koppelnde Ballon gewährleistet, dass die Bypass-Einheit in dislokationssicherer Weise fixiert über die Speiseröhre in den Magen und von dort in ihre trans-pylorische Ankerposition gebracht werden kann. Die beschriebene Kopplungstechnik wird ebenfalls in der US 10,667.936 B2 behandelt bzw. dort weiter ausgeführt.

In der WO 2016/067087 A2 wird das koppelnde Element im Anschluss an die Befüllung der trans-pylorisch retinierend platzierten Baueinheit entspannt bzw. entleert und das somit freigegebene Endoskop weiter durch das Lumen der Bypass-Vorrichtung hindurch, in das Duodenum vorgeschoben. Bei diesem Stand der Technik erfolgt die vollständige axiale Entfaltung bzw. Ausrollung des Bypass-Schlauches über das Treitzsche Band hinweg mittels eines Drahtes, der mit dem distalen Ende der Schlauchfolie verbunden ist, und über den Arbeitskanal des Endoskopes geführt wird bzw. über die Spitze des Endoskops hinaus ausgefahren wird und so die Bypass-Schlauchfolie mit sich nimmt. Nach dem Erreichen der Zielposition des Folienschlauches wird der Draht in den Endoskopschaft zurückgezogen bzw. aus dem Endoskop entfernt. Anschließend wird der Endoskopschaft aus dem entfalteten Folienschlauch bis in den Magen zurückgezogen. Über den nun freien Arbeitskanal des Endoskops wird sodann ein schneidendes, zangenartiges Werkzeug über den Arbeitskanal des Endoskops bis in den Magen eingeführt, um damit die zuleitenden Schlauchleitungen zu den Ballonkomponenten der Durchleitungs-Einheit zu kappen. Abschließend wird das Endoskop vollständig aus dem Patienten entnommen.

Die Anwendung der in der WO 2016/067087 A2 beschrieben Vorrichtung hat sich in der Praxis zwar als praktikabel dargestellt, jedoch kommt es beim Zurückziehen des Endoskopschaftes aus der entfalteten, auf seine gesamte Länge ausgestreckten Schlauchfolienkomponente der Bypass-Vorrichtung auch bei diesem Verfahren, bedingt durch die in Summe zu hohen Reibungs- und Adhäsionswirkungen, zu unerwünschten Retraktionen bzw. magenwärts gerichteten Mitnahmen der Schlauchfolie.

Die vorliegende Erfindung strebt daher nach einem technischen Lösungsweg für das Problem einer Applikation bzw. intrakorporalen Einbringung und Positionierung einer trans-duodenalen Bypass-Vorrichtung, die weitestgehend unabhängig von einem Endoskop sein soll.

Der erfindungsgemäße Lösungsweg basiert stattdessen auf einem gekoppelten Verbund eines spezifisch ausgeführten, eine sichere Führung der Applikation von außerhalb des Körpers gewährleistenden Positionierungskatheters mit einer dazu baulich und funktionell kongruenten Bypass-Einheit.

Insbesondere beschreibt die Erfindung bauliche Merkmale und durch diese Merkmale ermöglichte, spezifische Funktionsweisen, die es gestatten, Adhäsions- und Reibungseffekte zwischen den im Inneren des Folienschlauches geführten, positionierend wirkenden Schaftanteilen der Positionierungs-Einheit und der Innenfläche des Folienschlauches in vorteilhafter, Retraktions- bzw. Mitnahmeeffekte weitestgehend vermeidender Weise zu reduzieren.

Die vorgestellte Ausführungsform der im Körper verweilenden Bypass-Einheit ist hinsichtlich ihrer bevorzugten Bauweise bzw. Ausstattung mit Ballonkomponenten relativ konkret. Sie weist bevorzugt einen einzigen, diskoid ausgeformten Ballon für die post-pylorische Platzierung im Bulbus duodeni sowie eine doppelte, konzentrisch einhausende Ballonanordnung für die Positionssicherung auf der prä-pylorischen, gastrischen Seite auf. Der innere Ballon der Ballonanordnung hat dabei eine redundant sichernde Funktion, die gewährleistet, dass beim Verlust des äußeren Ballons bzw. dessen retinierender Funktion, eine duodenal gerichtete Dislokation der trans-pylorischen Durchleitungs-Einheit, mit der Folge eines akuten Verschlusses des duodenalen Lumens, vermieden werden kann.

Das den Schließmuskel aufnehmende Segment zwischen den duodenal- und magenseitigen Ballons weist in der bevorzugten Ausführungsform nur ein zentral durchleitendes, schlauch-artiges Segment auf, das dem Pylorus, ohne einen weiteren, zwischen Schlauch und Pylorus liegenden Ballonfolienanteil, direkt exponiert ist.

Die vorliegende Bypass-Einheit verfügt für eine einfache, besonders niederkomplex konstruierte, verlustfreie Befüllmöglichkeit der duodenal- und magenseitig angeordneten Ballonkomponenten. Hierfür kommen spezifisch ausgeführte, ringartige Dichtungskomponenten zum Einsatz. Diese liegen den zuleitenden bzw. befüllenden Öffnungen zum Binnenraum der Ballons unter elastischer Eigenspannung in flächig dichtender Weise auf. Der jeweilige Ventilring besteht bevorzugt aus einem Material mit besonderen, nicht ermüdenden dauerelastischen Eigenschaften, wie diese beispielsweise durch Polyurethane oder Silikone ermöglicht werden. Die jeweiligen Komponenten werden bevorzugt im Spritzgussverfahren hergestellt, um eine größtmögliche zirkumferenzielle Konstanz der Wandungsstärke der Ringe, eine größtmögliche Ebenheit der dichtend wirkenden Flächen sowie eine Konstanz der elastischen Eigenschaften zu gewährleisten. Die Oberfläche der jeweiligen, die Ringe tragenden Schaftkomponenten weisen bevorzugt einbettende, zirkulär verlaufende Strukturen auf, die die Ringe mulden-artig fassen und in der erforderlichen, dichtend wirkenden Position über den Zuleitungsöffnungen halten.

Für ein optimiertes trans-duodenales Ausrollen und/oder Entfalten der Folienschlauchkomponente der Bypass-Einheit kann die Schlauchkomponente am distalen Ende mit einer schleifen- bzw. henkelartigen Formation versehen sein, welche direkt von der distalen Spitze des jeweiligen, die Folie ins Duodenum transportierenden Schaftkörpers der Positionierungs-Einheit aufgenommen wird.

Eine solche, henkelartige Formation weist vorzugsweise eine apikale bzw. mittige Stanzung auf, durch welche ein Führungsdraht hindurchgeführt werden kann. Somit kann der Folienschlauch beim Vorschub der Positionierungs-Einheit mit seinem distalen, freien Ende voran in das Duodenum eingeführt bzw. durch das Duodenum hindurchgeführt werden.

In entsprechender Weise kann die henkelartige Formation auch durch eine geeignete Widerlagerkomponente aufgenommen und transportiert werden, die an der Spitze einer separaten Kathetereinheit angeordnet ist, welche bei optionalen Bauformen durch den Schaftkörper der Positionierungs-Einheit geführt werden kann und besondere, der duodenalen Passage angepasste Biegeeigenschaften aufweist.

Um eine möglichst problemlose Retraktion der Positionierungs-Einheit aus der Bypass-Einheit zu ermöglichen, sollte die Summe der adhäsiven Wirkungen der äußeren Wandung des Folienschlauches zur ihr exponierten Darmschleimhaut die Summe der adhäsiven und reibenden Wirkungen zwischen der inneren Wandung des Folienschlauches und der in ihr bewegten Schaftanteile der Positionierungs-Vorrichtung überschreiten, so dass beim Zurückziehen des distalen Anteils der Positionierungseinheit aus dem duodenal entfalteten Folienschlauch der Bypass-Einheit eine magenwärts gerichtete Mitnahme des Schlauches vermieden wird.

Die adhäsiven und reibenden Wirkungen zwischen der inneren Wandung des Folienschlauches und des in ihr bewegten Schaftanteils der Positionierungs-Vorrichtung können durch die Einbringung von reibungs- und adhäsionsreduzierenden Substanzen in den Binnenraum des Folienschlauches reduziert werden. Die Zuleitung reibungs-reduzierender Substanzen kann insbesondere auch durch deren kontinuierliche Zuführung während des Retraktionsvorganges erfolgen.

Die bauliche Ausführung der Erfindung ermöglicht ferner, dass die adhäsiven Wirkungen zwischen der äußeren Wandung des Folienschlauches und der ihr exponierten Darmschleimhaut durch eine passagere Beaufschlagung des Binnenraumes des Folienschlauches mit einem moderaten Druck erhöht oder maximiert werden. Die so hergestellte, vollständige radiale Entfaltung des Folienschlauches innerhalb des Darmlumens schmiegt die gesamte äußere Oberfläche des Schlauches flächig an die Schleimhaut an, wodurch ein größtmöglicher Adhäsionseffekt hergestellt wird.

Um eine entsprechende Befüllung zu gewährleisten, kann die Positionierungs-Einheit mit Funktionen bzw. Komponenten ausgestattet sein, die sowohl einen proximalen als auch (zusätzlich) einen distalen, passager wirkenden Verschluss des Folienschlauchlumens ermöglichen, so dass sich ein allseitig abgeschlossener Lumenabschnitt realisieren lässt, der sodann bspw. mit einem erhöhten Druck beaufschlagt werden kann.

Die Erfindung empfiehlt, dass die im Patienten verbleibende Bypass-Einheit eine trans-pylorische Schaftkomponente aus einem elastischen Polymer aufweist, so dass sie sich der lokalen Anatomie und Motilität des Pylorus anpassen kann.

Bevorzugt wird die Schaftkomponente der Bypass-Einheit durch Blasformen hegestellt, bspw. aus einem extrudierten Schlauchrohling. Dabei ist es möglich, in die Schaftkomponente der Bypass-Einheit eine Korrugation einzuformen, bspw. um deren elastische Eigenschaften zu optimieren.

Andererseits kann die die Schaftkomponente der Bypass-Einheit auch durch Spritzguss hergestellt werden. Dabei besteht die Möglichkeit, den Schaft der Bypass-Einheit mit schaft-integrierten Lumina für die Befüllung der vom Schaft getragenen Ballonkomponenten zu versehen.

Die trans-pylorische Baugruppe der Bypass-Einheit trägt auf ihrem distalen Segment eine Ballonkomponente für die Positionierung im Bulbus duodeni. Diese besteht bevorzugt aus thermoplastischem Polyurethan (TPU). Die distale Ballonkomponente wird in der bevorzugten Ausführungsform bereits bei der Herstellung auf ihr erforderliches Arbeitsmaß oder darüber hinaus ausgeformt, so dass für ihre Befüllung bzw. für die Einstellung des erforderlichen in situ Arbeitsdurchmessers keine kraftintensive Dehnung der Ballonhülle erforderlich ist. Die duodenal platzierte Ballonkomponente wird bevorzugt mit einem Volumen befüllt, das 80 bis 90% des außerhalb des Körpers spannungslos frei entfalteten Volumens der auf dem Schaft montierten Ballonkomponente entspricht. Die Ballonwandung verbleibt somit in einem spannungslos schlaffen, ungedehnten Zustand. Die Entfaltung bzw. Platzierung des Ballons innerhalb des Bulbus duodeni kann auf diese Weise mit besonders schonenden, atraumatischen Fülldrucken erfolgen, die den jeweils im Bulbus duodeni herrschenden physiologischen Druckwerten nahezu entsprechen. Die duodenale Ballonkomponente wird bevorzugt mit flüssigen Medien befüllt.

Die trans-pylorische Baugruppe verfügt ferner über eine proximale, magenseitig platzierte Ballonanordnung. Sie umfasst vorzugsweise zwei Ballonkomponenten, die konzentrisch ineinander angeordnet sind, so dass durch den inneren Ballon eine sichernde Redundanz gewährleistet wird, die bei einem strukturellen Versagen oder einer sonstigen Entleerung des äußeren Ballons ein Abgleiten der Bypass-Einheit in den Darm ausschließt. Dabei besteht die innere der beiden konzentrischen Ballonkomponenten vorzugsweise aus einem einlagigen thermoplastischen Polyurethan TPU. Sie wird bevorzugt mit Wasser oder sonstigen körperverträglichen Flüssigkeiten, beispielsweise auch ölbasierten Lösungen, befüllt.

Die äußere Ballonkomponente besteht bevorzugt aus einem zwei-lagigen Material, wobei die Außenseite der Ballonhülle in besonderer Weise säure- bzw. hydrolysebeständig ist, so dass ein Verbleib im sauren Milieu des Magens über einen längeren Zeitraum von bis zu 12 Monaten möglich ist. Hier kommen Polyamid- oder Pebax-basierte Polymere zum Einsatz. Die Herstellung der mehrlagigen Ballonkomponente erfolgt bevorzugt durch Blasformung aus einem entsprechend mehrlagig extrudierten Schlauch. Die innere Lage besteht vorzugsweise aus einem thermoplastischen Polyurethan TPU, wobei höhere Shore-Härten wie zum Beispiel 95A oder 55D bis 60D verwendet werden können, die sich durch eine höhere Säurestabilität ausweisen.

Neben dem äußeren gastrischen Ballon, kann auch der duodenale Ballon aus einem entsprechenden, zwei-lagigen Material hergestellt werden.

Die Ballons der trans-pylorischen Baugruppe werden über langstreckige, trans-ösophageal geführte Schlauchzuleitungen befüllt, die am proximalen Ende der Baugruppe mit dieser fix verbunden sind und die Befüllung der Ballonkomponenten von extrakorporal erlauben. Die Schlauchzuleitungen sind baulich derart ausgeführt, dass sie mit endoskopisch applizierten Schneidewerkzeugen im Bereich der gastrischen Stirnseite der Bypass-Einheit durchtrennt werden können.

Sämtliche Ballonkomponenten der trans-pylorischen Baugruppe werden bevorzugt mit flüssigen Medien befüllt, wobei alle Ballons bevorzugt, wie zuvor beschrieben, auf ihr Arbeitsmaß oder darüber hinaus ausgeformt bzw. vorgeformt sind und nur partiell befüllt werden, so dass die Ballons einen spannungslosen, nichtgedehnten Zustand annehmen, wodurch insbesondere der Tragekomfort des Patienten verbessert bzw. peri-pylorische Irritationen vermindert werden.

Die Befüllung der Ballons kann jeweils separat über individuelle Zuleitungen erfolgen. Die Befüllung kann alternativ derart angelegt sein, dass zwei der drei Ballons, beispielsweise der duodenale und der innere gastrische Ballon, über eine Leitung gemeinsam befüllt werden.

Die Erfindung sieht ferner eine Befüllung vor, wobei der duodenale und der innere gastrische Ballon jeweils separat befüllt werden, und wobei der äußere gastrische Ballon passiv dem inneren Ballon bei dessen Befüllung folgen und selbst über keine eigene Zuleitung bzw. Fülloption verfügen kann.

Während der Lagerung, der trans-ösophagealen Einbringung und der trans-pylorischen Platzierung der Bypass-Einheit wird die den Bypass bewirkende bzw. gewährleistende Schlauchfolienkomponente auf eine kompakte Länge von beispielsweise etwa 3 bis 5 cm gerafft. Um den gerafften Zustand der Folie während der Lagerung der Bypass-Vorrichtung aufrechtzuerhalten bzw. die geraffte Folie aufzunehmen, kann hierfür ein spezieller aufnehmender Konus verwendet werden, der in das distale Ende der trans-pylorischen Schaftkomponente eingesteckt ist. Er wird unmittelbar vor der Applikation entnommen.

Sämtliche, im Rahmen der Erfindung beschriebenen baulichen Ausführungsformen basieren bevorzugt auf der initialen Anlage eines endoskopisch platzierten, schienend wirkenden Führungsdrahtes. Das Standardmaß von Führungsdrähten in der klinischen Endoskopie beträgt 0,038 inch, was 0,96 mm entspricht. Die Drähte sind in der Regel mit einer flexiblen Spitze ausgestattet und gewährleisten, endoskopisch geführt bzw. über den Arbeitskanal eines Endoskopes eingebracht, eine relativ gefahrlose Positionierung des Drahtes bis etwa 30 cm in das Jejunum.

Die vorliegende Erfindung separiert den Vorgang der trans-ösophagealen und trans-pylorischen Passage, sowie die trans-duodenale Ausrollung der Bypass-Schlauchfolie weitgehend vom Einsatz endoskopartiger Geräte. Endoskope werden im Rahmen der Applikation lediglich für das initiale Einbringen eines schienenden, den gekoppelten Verbund der Bypass- und Positionierungs-Einheit aufnehmenden Führungsdrahtes verwendet. Darüber hinaus haben Endoskope lediglich eine, alle Teilschritte der Applikation überwachende Funktion, in dem sie eine magenseitige Sicht auf den Pylorus und die dort platzierte trans-pylorische Baugruppe ermöglichen. Endoskope kommen zudem bei der Durchtrennung der Zuleitungsschläuche zu den Ballonkomponenten zum Einsatz.

Die Bypass-Einheit wird in spezifischer Weise mit der Positionierungs-Einheit über eine in der Vorrichtung zwischen den Einheiten integrierte Baugruppe gekoppelt. In dieser verschiebestabil gekoppelten Anordnung wird der Verbund der Einheiten über den vorausgehend endoskopisch angelegten, schienend wirkenden Führungsdraht hinweg, über den Mund und Rachen, trans-ösophageal in den Magen vorgeschoben.

Der Schaftkörper der katheter-artigen Positionierungs-Vorrichtung verfügt hierfür über die entsprechende Steifigkeit, um den Widerstand sowohl bei der ösophagealen als auch bei der sich anschließenden pylorischen Passage zu überwinden. Der Schaftkörper hat einen bevorzugten äußeren Durchmesser von ca. 8 bis 12 mm und kann daher vom Anwender manuell gut gegriffen und geführt werden. Insbesondere werden extrakorporale Drehbewegungen bzw. Rotationen des Schaftes auf den distalen Schaftanteil verlässlich übertragen. Der über den Schaftkörper vermittelte Vorschub der Vorrichtung erfolgt, im Verbund mit dem im zentralen Schaftlumen verlaufenden Führungsdraht, ohne relevante Stauchung und Knickung.

Eine weitere grundsätzliche bauliche Maßnahme zur Reduktion adhäsiver und reibungsbedingter Retraktion der duodenal entfalteten Schlauchfolie besteht in der baulichen Verkleinerung der Kontaktfläche zwischen der jeweiligen, trans-duodenal manövrierten Schaftkomponente der Positionierungs-Einheit und der Innenfläche der Folienschlauchkomponente. Bei einer endoskopischen Positionierung wirken sich Schaftdurchmesser von ca. 10 mm, im Verhältnis zu Innendurchmessern der Schlauchfolie von ca. 20 bis 30 mm, infolge der Summe der dann wirkenden Reibungs- und Adhäsionseffekte, in der praktischen Anwendung nachteilig aus. Die vorliegende Erfindung verwendet dagegen bevorzugt Ausführungsformen, wobei der äußere Durchmesser des Schaftkörpers der Positionierungs-Einheit nur etwa 3 bis 7 mm beträgt, vorzugsweise zwischen 4 und 6 mm, also etwa 1/4 bis 1/5 des Durchmessers der trans-duodenal applizierten Schlauchfolie, die im vollständig radial entfalteten Zustand einen Durchmesser von etwa 20 bis 30 mm aufweist.

Als weitere Möglichkeit zur Reduzierung der adhäsiven und reibenden Wirkungen zwischen der inneren Wandung des Folienschlauches und der in ihr bewegten Schaftanteile der Positionier-Vorrichtung kann im Moment des Manövrierens bzw. der gesteuerten relativen Bewegung des Schaftkörpers innerhalb des Folienschlauches ein Medium wie Wasser oder Luft in den Binnenraum des Schlauches infundiert oder insuffliert werden, so dass sich der Folienschlauch mit einem geringen Druck innerhalb des Duodenums teilweise oder vollständig aufrichtet, wodurch sich der Folienschlauch von der Positionier-Vorrichtung in kontaktreduzierender Weise abhebt.

Um diesen Effekt zu maximieren, sieht die Erfindung eine Anwendungsoption vor, die es ermöglicht, dass sich die äußere Oberfläche des Folienschlauches vollständig flächig an die Darmschleimhaut anlegt und somit ein größtmöglicher Adhäsionseffekt der Folie zum Darm hergestellt wird. Der Binnenraum innerhalb des Folienschlauchs wird hierzu an seinen proximalen und distalen Enden verschlossen bzw. so weit gedichtet, dass er mit einem moderaten Druck beaufschlagt werden kann, bspw. in der Größenordnung von 10 bis 50 mbar.

Die Applikation der Bypass-Einheit kann dahin gehend optimiert werden, dass alle zuvor beschriebenen Maßnahmen für eine Reduzierung der adhäsiven und reibenden Wirkungen zwischen der inneren Wandung des Folienschlauches und des in ihr bewegten Schaftanteils der Positionier-Vorrichtung bei der Anwendung kombiniert werden.

Die Erfindung nimmt optional eine Bauweise einer Bypass-Vorrichtung auf, wie diese in der WO 2016/067087 A2 beschrieben ist. Dort wird eine für die dynamisch adaptiv dichtende und atraumatisch organverträgliche, trans-pylorische Fixierung besonders vorteilhafte Bau- und Funktionsweise beschrieben, wobei die Vorrichtung mindestens eine Ballonkomponente aufweist, die derart auf einer trans-pylorisch durchleitenden Schaftkomponente aufgebracht ist, dass die Ballonkomponente bei der Befüllung bzw. Beaufschlagung mit Fülldruck eine zum Pylorus hin gerichtete Rollbewegung vollzieht, und gleichzeitig die radiale Expansion, insbesondere des duodenalseitigen Ballonkorpus zu den anliegenden Geweben und Strukturen so weit wie möglich begrenzt werden kann. Die im Rahmen der vorliegenden Erfindung beschriebene Bypass-Vorrichtung kann das Merkmal der axial gerichteten, dichtend und fixierend wirkenden Ballon- bzw. Widerlagerfunktion optional aufweisen.

Der Schaftkörper der Positionierungs-Einheit kann ferner eine Bauweise aufweisen, wobei der katheter-artige Schaftkörper durchgängig von oral bis jejunal, ein durchgängiges Profil und/oder einen durchgängigen Durchmesser aufweist, und/oder eine identische Anordnung der im Schaft integrierten Lumina, und/oder ein einheitliches Schaftmaterial.

Andererseits kann der Schaftkörper der Positionierungs-Einheit auch aus mehreren, unterschiedlich bemessenen Segmenten aus verschiedenen Materialien bestehen, beispielsweise also mehrere Segmente mit jeweils verschiedenen, ihrer Aufgabe angepassten Biege- bzw. Steifigkeitseigenschaften aufweisen. Hierbei weisen die proximalen, in den Magen und den Pylorus reichenden Segmente relativ größere Durchmesser auf als die distalen Segmente, welche bei der Applikation in das Duodenum und Jejunum vorgeschoben werden.

Die Bauweise des Schaftkörpers kann grundsätzlich auch konzentrisch zueinander angeordnete bzw. ineinander verlaufende Schaftkomponenten umfassen, wobei durch ein proximales, äußeres, im Durchmesser größeres, relativ rigides, den knickstabilen trans-ösophagealen und trans-pylorischen Vorschub gewährleistendes Segment ein distales, inneres, relativ flexibles, axial biegbares Segment verläuft.

Das distale Schaftsegment weist Biegeeigenschaften auf, welche die Passage der typischen, C-förmigen Windung des Duodenums und den flexur-artigen Übergang über das Treitzsche Band erleichtern sollen. Elastische Rückstellkräfte bei axialer Biegung des distalen Schaftsegmentes sollen möglichst auch bei starken Biegungen um bis zu 180 Grad vermieden werden, wobei gleichzeitig beim axialen Vorschub des Segmentes keine ziehharmonika-artigen Stauchungseffekte auftreten sollten.

Das proximale Segment des Schaftkörpers der Positionierungs-Einheit ist bezüglich seines Durchmessers und der eingesetzten Materialien derart bemessen bzw. beschaffen, dass es auf seiner Außenfläche die jeweilige, zu applizierende Bypass-Einheit aufnehmen kann. Der Außendurchmesser des Schaftkörpers wird derart gewählt, dass er zum durchleitenden Kanal der trans-pylorischen Baugruppe einen Spaltraum ausbildet, in dem eine Kopplungs-Einheit platziert werden kann.

Eine weitere Ausführungsform der Vorrichtung kann baulich auf einem Schaftkörper der Positionierungs-Einheit basieren, der nicht über das obere Duodenum hinausreicht und keinen Innenkatheter aufnimmt, sondern wobei der Vorschub der Schlauchfolie durch eine ausschließlich draht-basierte Komponente erfolgt, die den vorderen, distalen Rand der Schlauchfolie bzw. die dort angebrachte henkelartige Struktur mittels eines am Draht angebrachten Werkzeugs oder Widerlagers aufnimmt und die Schlauchfolie so beim Vorschieben mit sich führt.

Ein solcher Führungsdraht erlaubt eine Weiterbildung der Erfindung dahingehend, dass gemeinsam mit dem Führungsdraht oder auch der endoskopischen Anlage des Drahtes nachfolgend, ein zusätzlicher kleinlumiger Katheter eingebracht wird, der die erforderlichen Biege- bzw. Führungseigenschaften des Drahtes bei der Applikation der Vorrichtung bzw. ihrer Einheiten und deren Segmente im Körper optimiert. Der entsprechende kleinlumige, modifizierend wirkende Katheter kann optional mit einer verschließend und ankernd wirkenden, von extrakorporal befüllbaren Ballonkomponente ausgestattet sein. Der befüllte Ballon stabilisiert den Verbund von Katheter und Draht in seiner jeweiligen, endständigen Lage. Der Ballon kann optional auch zum lumendichtenden Verschluss des Folienschlauches zur Befüllung des Schlauchbinnenraumes oder der Beaufschlagung mit einem Druck verwendet werden.

Die distale Spitzenformation der Positionier-Einheit kann eine atraumatisch, beispielsweise gerundete Kegelform aufweisen, die beim trans-luminalen Vorschub eine Verletzung der Organwandung verhindert.

Der Schaftkörper der Positionier-Einheit kann eine Gesamtlänge von mindestens 150 cm aufweisen, um so, bei einer Führung von extrakorporal, die Bereiche aboral des Treitzschen Bandes erreichen zu können. Der zu verwendende Führungsdraht sollte die Länge der Positionier-Einheit um mindestens 150 bis 200 cm überschreiten.

Ferner richtet sich die Erfindung - unabhängig von einem Gesamtsystem mit den übrigen erfindungsgemäßen Bestandteilen - auch auf eine trans-duodenale Bypass-Vorrichtung als solche, wobei jene umfasst: (i) eine trans-pylorische Baugruppe in Form einer ballon-basierten Einheit für die langfristige, atraumatisch organverträgliche Fixierung der Bypass-Vorrichtung im Bereich des Schließmuskels des Magenausgangs (Pylorus), sowie (ii) eine trans-duodenale Baugruppe in Form einer Folienschlauch-Einheit für die bypass-artige, trans-duodenale Durchleitung von Mageninhalt in die höheren Anteile des Jejunums, wobei die im Patienten platzierte Bypass-Vorrichtung von der zum Einbringen verwendeten Positionier-Vorrichtung von extra-korporal kontrollierbar entkoppelt werden kann.

Erfindungsgemäß kann dabei weiter vorgesehen sein, dass die befüllenden Zuleitungen zu retinierend wirkenden, duodenal- und magenseitig angeordneten Ballonkomponenten über ringartige, der Oberfläche der durchleitenden Schaftkomponente der Durchleitungs-Einheit der Bypass-Vorrichtung unter elastischer Spannung anliegende Dichtungskomponenten angeschlossen sind.

Außerdem kann eine solche Bypass-Vorrichtung eines, mehrere oder alle Merkmale der Bypass-Einheit gemäß den beigefügten Patentansprüchen aufweisen.

Bspw. könnten die ringförmigen Dichtungskomponenten aus einem Material mit dauerhaften, nicht ermüdenden elastischen Dehnungseigenschaften bestehen, bspw. aus Polyurethan oder Silikon, und/oder an der (den) jeweilige(n), der betreffenden Ballonkomponente zugeordneten Öffnung(en) in der Schaftkomponente der Bypass-Vorrichtung in dichtender Weise aufliegen, vorzugsweise die Ränder der betreffenden Öffnung(en) allseits flächig überlappend. Bspw. könnten dabei die jene ringförmigen Dichtungskomponenten tragenden Abschnitte der Schaftkomponente der Bypass-Vorrichtung an der jeweiligen Außenseite der Schaftkomponente jeweils zwei zirkulär umlaufende Erhebungen aufweisen, welche einen gegenseitigen Abstand aufweisen, welcher der Breite der betreffenden, ringförmigen Dichtungskomponente entspricht, und die betreffende, ringförmige Dichtungskomponenten in einbettender Weise zwischen sich sichernd aufnehmen.

Ferner ist es möglich, dass die Schaftkomponente einer solchen Bypass-Vorrichtung eine Schaftinnenstruktur aus einem elastischen Polymer aufweist, bevorzugt blasgeformt oder spritzgegossen, vorzugsweise mit eingeformten Korrugationen und/oder mit integrierten Öffnungen und/oder Lumina für die Befüllung von an der Außenseite der Schaftkomponente vorgesehenen Ballonkomponenten.

Eine solche Bypass-Vorrichtung kann eine distale Ballonkomponente für die Positionierung im Bulbus duodeni aufweisen, bevorzugt aus TPU, die vollständig ausgeformt sein kann, und in schlaff befülltem Zustand auf ca. 80 bis 90 % ihres vorgeformten bzw. frei entfalteten Volumens aufgebläht wird, und bevorzugt mit einem Gas befüllt wird, bspw. mit Luft.

Darpber hinaus kann eine solche Bypass-Vorrichtung eine proximale Ballonanordnung für die Positionierung im Magen aufweisen, bspw. mit zwei Ballons, die vorzugsweise ineinender eingehaust sind.

Dabei kann der innere proximale Ballon bevorzugt aus einer einzigen Lage bestehen, insbesondere aus einem thermoplastischen Polyurethan (TPU).

Der distale und/oder der äußere proximale Ballon können jeweils aus einem zwei-lagigen Material bestehen, das bevorzugt säurebeständig ist.

Während der innere proximale Ballon bevorzugt mit einer Flüssigkeit befüllt wird, kann der äußere proximale Ballon bevorzugt mit einem Gas befüllt werden, bspw. mit Luft, oder unbefüllt bleiben.

Andererseits kann die Schlauchfolie der trans-duodenalen Baugruppe (TDG) riner solchen Bypass-Vorrichtung, vorzugsweise im Bereich ihres distalen Endes, eine Schlinge oder eine henkel- oder schleifenartige Formation aufweist, womit sie sich leichter entfalten lässt. Diese jeweilige Schleifen- oder Henkelformation kann eine apikale Stanzung für die Durchführung einer Führungsdraht-Komponente oder fingerartigen Aufnahmekomponente der Spitze einer Positionier-Vorrichtung aufweisen.

Schließlich besteht auch die Möglichkeit, die Außenseite einer Folienschlauchkomponente mit einer adhäsiven und/oder reibungserhöhenden Beschichtung zu versehen, bspw. mit einem Gel od. dgl.

Ferner schlägt die Erfindung - ebenfalls unabhängig von einem Gesamtsystem mit den übrigen erfindungsgemäßen Bestandteilen - eine trans-ösophageale Positionier-Vorrichtung für die endoskopisch unterstützte, trans-ösophageale Einbringung und trans-pylorische Fixierung einer trans-duodenalen Bypass-Vorrichtung vor, wobei die trans-ösophageale Positionier-Vorrichtung einen proximalen Anteil für die Einbringung der Bypass-Vorrichtung in den Magen und deren Fixierung im Bereich des Schließmuskels des Magenausgangs (Pylorus) bzw. im dem Pylorus anatomisch angrenzenden Bereich aufweist, sowie ggf. einen distalen Anteil für die Entfaltung oder Entraffung einer Schlauchfolie für die bypass-artige, trans-duodenale Durchleitung von Mageninhalt in die höheren Anteile des Jejunums, wobei die Positionier-Vorrichtung von der platzierten Bypass-Vorrichtung von extra-korporal kontrollierbar entkoppelt werden kann.

Eine solche, erfindungsgemäße Positionier-Vorrichtung erlaubt es, über die im Rahmen der Erfindung beschriebenen spezifischen Bauformen der Bypass-Einheit hinaus, verschiedenste andere Bauformen von trans-pylorischen Durchleitungs- und Fixierungs-Einheiten aufzunehmen und in das duodenale Lumen einzuführen und trans-pylorisch zu platzieren sowie deren Schlauchfolien-Einheit in das jejunale Lumen auszurollen oder zu entfalten sowie im Anschluss daran die Positionier-Einheit komfortabel bzw. mit einer nur geringen retrograden Mitnahmewirkung der entfalteten Schlauchfolie wieder zurückzuziehen.

Dabei liegt es im Rahmen der Erfindung einer solchen Positionier-Vorrichtung als solche, dass bei der Retraktion des Anteils einer zur Platzierung im Patienten verwendete Positionier-Vorrichtung aus dem duodenal und jejunal entfalteten Folienschlauch der Bypass-Vorrichtung eine Mitnahme bzw. nach oral gerichtete Raffung des Folienschlauches vermieden wird, indem die Summe der adhäsiven und/oder reibungsverursachenden Wirkungen zwischen der äußeren Wandung des Folienschlauches und der ihr exponierten Darmschleimhaut die Summe der adhäsiven und/oder reibenden Wirkungen zwischen der inneren Wandung des Folienschlauches und des in ihr bewegten Schaftanteils der Positionier-Vorrichtung überschreitet.

Ferner kann eine solche Positionier-Vorrichtung eines, mehrere oder alle Merkmale der Positionier-Einheit gemäß den beigefügten Patentansprüchen aufweisen.

Bspw. könnte die Positionier-Vorrichtung ein zentrales Lumen aufweisen, um entlang eines zuvor mittels eines Endoskops verlegten Führungsdrahtes trans-ösophageal vorgeschoben zu werden.

Außerdem könnten die adhäsiven und/oder reibenden Wirkungen zwischen der inneren Wandung der Schlauchfolie einer Bypass-Vorrichtung und des in ihr bewegten Schaftanteils einer erfidnungsgemäßen Positionier-Vorrichtung durch eine Einreichtung zur Einbringung von reibungs- und/oder adhäsionsreduzierenden Substanzen in den Binnenraum des Folienschlauches reduziert werden, und/oder durch eine Einrichtung zur Insufflation von Wasser oder Luft in den Binnenraum des Folienschlauches, welche die adhäsiven und reibenden Wirkungen zwischen der inneren Wandung des Folienschlauches und des in ihr bewegten Schaftanteils der Positionier-Einheit reduziert.

Eine weitere Maßnahme, um die adhäsiven und reibenden Wirkungen der äußeren Wandung einer Schlauchfolie einer Bypass-Vorrichtung zur ihr exponierten Darmschleimhaut zu erhöhen, ist eine passagere Beaufschlagung eines dortigen, allseits abgeschlossenen Binnenraumes innerhalb der Schlauchfolie einer Bypass-Vorrichtung mit einem moderaten Druck, wodurch sich im Zustand der vollständigen Entfaltung der Schlauchfolie innerhalb des Darmlumens die äußere Oberfläche der Schlauchfolie vollständig flächig an die Schleimhaut anlegt und somit ein größtmöglicher Adhäsionseffekt hergestellt wird.

Dies gelingt durch eine Einrichtung im Rahmen der Positionier-Vorrichtung zur Beaufschlagung eines abgeschlossenen Binnenraumes innerhalb des Folienschlauchs mit einem moderaten Druck, und/oder durch eine Einrichtung zum passageren Verschluss des proximalen und des distalen Endes des Folienschlauches, damit der solchermaßen abgeschlossene Binnenraum innerhalb des Folienschlauchs mit einem moderaten Druck beaufschlagt werden kann.

Letzteres lässt sich dadurch erreichen, dass eine distale Schaftkomponente der Positionier-Vorrichtung wenigstens einen distalen Ballon trägt, welcher ankernd wirken kann und/oder das distale Lumen der Folienschlauch-Komponente verschließen kann, und/oder welcher eine henkelartige Struktur am distalen Ende der Schlauchfolie einer Bypass-Vorrichtung nach aboral führen bzw. verschieben kann.

Die Schaftkomponente einer erfindungsgemäßen Positionier-Vorrichtung kann aus einem einzigen Element mit durchgehendem Profil bestehen, oder aus mehreren Segmenten mit unterschiedlichen Profilen zusammengesetzt sein.

Dieser Erfindungsaspekt lässt sich dadurch weiter verfolgen, dass ein Segment am distalen Ende der Schaftkomponente der Positionier-Vorrichtung eine höhere Biegsamkeit bzw. Flexibilität oder Elastizität aufweist als ein oder mehrere, proximal dazu angeordnete Segmente, und/oder dadurch, dass zwei Segmente der Schaftkomponente der Positionier-Vorrichtung mit unterschiedlichen Profilen teleskopartig ineinander angeordnet sind und relativ zueinander verschiebbar sind.

Die folgenden Abbildungen beschreiben grundsätzliche Bau- und Funktionsweisen der vorliegenden Erfindung anhand von verschiedenen, spezifischen, technischen Ausführungsformen. Hierbei zeigt:
- Fig.1: eine erfindungsgemäße Vorrichtung, bestehend aus einer im Körper verweilenden, trans-pylorisch platzierten Bypass-Einheit, einer applikationsunterstützenden Positionierungs-Einheit sowie einer, die beiden Einheiten reversibel koppelnden Einheit;
- Fig.2: eine bauliche Ausführungsform, wobei das distale Segment des Schaftkörpers der Positionierungs-Einheit mit einer welligen, die Biegeeigenschaften modifizierenden Profilgebung versehen ist;
- Fig.3a: eine, den Verbund der Bypass- und der Positionierungs-Einheit herstellende Kopplungs-Einheit, ausgeführt als baulich separierter, folienbasierter Hohlzylinder;
- Fig.3b: eine koppelnde Ballonkomponente, die fest auf dem Schaftkörper der Positionierungs-Einheit aufgebracht ist;
- Fig.3c: eine weitere koppelnde Ballonkomponente, die fest auf der Oberfläche des Schaftkörpers der Positionierungs-Einheit aufgebracht ist, oder frei lösbar im Spaltraum zwischen der Bypass- und der Positionierungs-Einheit eingefügt ist;
- Fig.4: eine Bauform einer Vorrichtung, bei der die Positionierungs-Einheit aus einem großlumigen proximalen Schaftsegment besteht und durch eine darin geführte, distale, frei verschiebliche, kleinlumige, die Schlauchfolie der Bypass-Einheit entfaltende, zusätzliche Katheter-Einheit ergänzt wird;
- Fig.4a: eine schleifenartige Formation für die Aufnahme des distalen Endes der trans-duodenal entfalteten Folienschlauch-Komponente, im Verbund mit einer die Folie jejunalwärts transportierenden, kleinlumigen Katheter-Einheit;
- Fig.4b: einen weiteren Verbund der distalen Anordnung der Folienschlauch-Komponente und einer die Folie jejunalwärts transportierenden Katheter- Einheit;
- Fig.5: eine lumenokkludierende Ballonkomponente für den dichtenden Verschluss im distalen Segment der Folienschlauch-Komponente;
- Fig.6: einen transversalen Querschnitt durch einen Abschnitt des Duodenums, mit einem darin flächig, adhäsionsmaximierend platzierten Bypass-Folienschlauch;
- Fig.7: spezifische bauliche Merkmale der im Körper verweilenden Bypass-Einheit;
- Fig.8: die schematische Anordnung der Vorrichtung und ihrer Einheiten im Magendarmtrakt; sowie
- Fig.9: eine in die Bypass-Einheit integrierte, messende Funktion, die den Abfluss von Mageninhalt in das Duodenum quantitativ erfasst, sowie optional die qualitative Zusammensetzung des Speisebreis ermittelt, und die gemessenen Werte an eine auswertende und darstellende, extrakorporale Systemeinheit in drahtloser Weise weiterleitet.

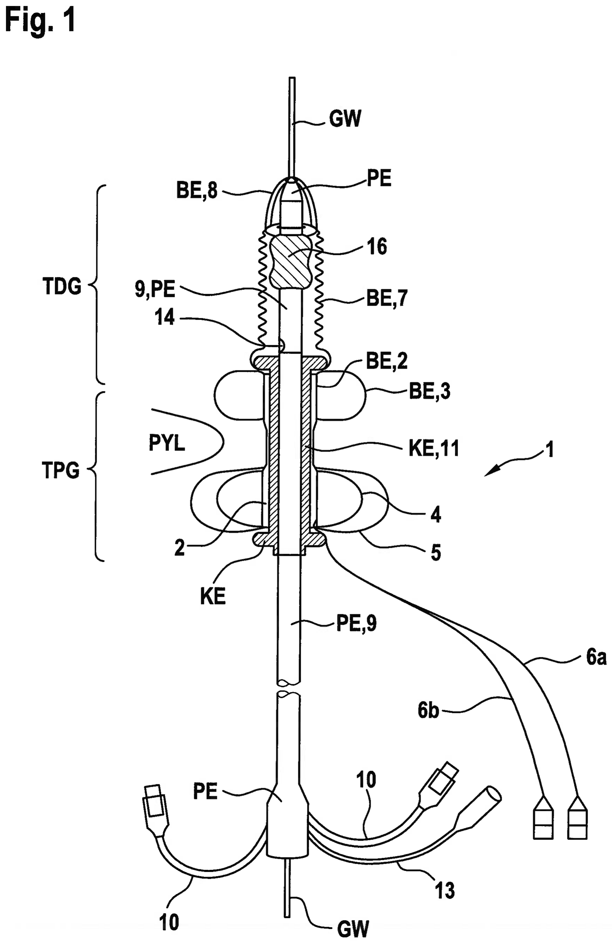

Fig.1 zeigt eine bevorzugte Ausführungsform einer erfindungsgemäßen Bypass-Vorrichtung 1, bestehend aus einer im Patienten verweilenden Bypass-Einheit BE, einer die trans-duodenale, trans-pylorische und trans-duodenale Anlage der Bypass-Einheit unterstützende Positionierung-Einheit PE, sowie einer Kopplungs-Einheit KE, die die Bypass-Einheit mit der Positionierungs-Einheit während der endoskopisch kontrollierten trans-ösophagealen und trans-pylorischen Einbringung der Vorrichtung miteinander dislokationssicher verbindet. Die Vorrichtung umfasst ferner einen Führungsdraht GW, der zu Beginn der Applikation, endoskopisch geführt, in Position gebracht wird bzw. über das Treitzsche Band hinweg, bis in das sich an das Duodenum anschließende Jejunum vorgeschoben wird.

Die verweilende Bypass-Einheit BE weist eine zentrale, schlauch- bzw. rohrartige Schaftkomponente 2 auf, die bevorzugt aus spritzgegossenem, sich elastisch verformendem und aufrichtendem, thermoplastischen Polyurethan (TPU) hergestellt ist. Die Schaftkomponente weist am distalen und proximalen Ende jeweils Flächen für die Fixierung von ausgeformten Ballonkomponenten 3, 4 und 5 auf. Die jeweiligen Flächen sind baulich derart verstärkt, dass sie den, in den jeweiligen Ballons herrschenden Fülldrucken widerstehen und eine lumenverengende Reduktion bzw. einen lumenverschließenden Kollaps des durchleitenden Kanals der Schaftkomponente ausschließen.

Die Befüllung der Ballonkomponenten erfolgt über kanalartige Strukturen, die vorzugsweise in die Wandung der Schaftkomponente integriert sind und am magenseitigen, proximalen Ende der Schaftkomponente in schlauchartige Zuleitungen 6a und 6b übergehen, die nach extrakorporal reichen und über die die Ballons mit geeigneten Medien, vorzugsweise nicht-kompressiblen Flüssigkeiten, befüllt werden können.

Die Ballonkomponenten 3, 4 und 5 bestehen vorzugsweise aus mehrlagig extrudiertem, durch Blasformung sphärisch oder diskoid ausgeformtem, folien-artigen Material, wobei die jeweils innere Folienlage vorzugsweise aus einem thermoplastischen Polyurethan besteht und die jeweilige äußere Lage vorzugsweise aus einem Polymer, mit besonders hydrolysestabilen Eigenschaften, wie beispielsweise polyamidbasierten oder polyamid-verwandten Materialien, wie z.B. Pebax, beschaffen ist. Die Fixierung der jeweiligen Ballonfolien auf der Schaftkomponente erfolgt dabei bevorzugt derart, dass jeweils die innere, polyurethanbasierte Lage der Ballons mit der Oberfläche der Schaftkomponente verbunden wird, wodurch bessere adhäsive Klebeeigenschaften erreicht werden können, als dies bei hydrolysestabilen Materialien in der Regel möglich ist. Insbesondere bietet sich bei der Anbindung der inneren TPU-Lage an eine ebenfalls TPU-basierte Schaftkomponente die Möglichkeit einer lösungsmittelbasierten Klebung, die auf säureempfindliche, potenziell brechende, adhäsive Kleber verzichtet.

Die Bypass-Einheit BE wird trans-pylorisch, also über den schließenden und öffnenden Muskel des Magenausgangs (Pylorus) hinwegreichend platziert. Sie weist zwei Baugruppen auf. Die trans-pylorische Baugruppe TPG, umfassend die Schaftkomponente 2, die Ballonkomponenten 3, 4 und 5, und deren schlauchartigen Zuleitungen 6a, 6b und ggf. auch 6c, sofern jeder der drei Ballonkomponenten separat befüllbar sein soll. Die Positionierung der Baugruppe erfolgt derart, dass der pylorische Schließmuskel PYL zwischen den konzentrisch angeordneten, magenseitigen Ballons 4, 5 und dem duodenalseitigen Ballon 3 zu liegen kommt.

Die trans-duodenale Baugruppe TDG der Bypass-Einheit BE umfasst einen großlumigen, folienartig dünnwandigen Schlauch 7 an dessen distales Ende eine Schlaufe 8 angebracht ist. Der Folienschlauch schließt sich direkt an das distale Ende der Schaftkomponente der trans-pylorischen Baugruppe an. Die Schlaufe ist am distalen, offenen Ende des Folienschlauchs henkelartig befestigt. Sie weist apikal eine lochartige Perforation auf, durch die der Führungsdraht GW der Vorrichtung geführt wird. Während die trans-pylorische Baugruppe die Bypass-Einheit in ihrer pylorischen Position vor duodenal- oder magenwärts gerichteter Dislokation sichert, gewährleistet die trans-duodenale Baugruppe die bypassartige Durchleitung des Mageninhaltes durch das Duodenum bis in das nachfolgende Jejunum.

Im zentralen, den Mageninhalt aufnehmenden und durchleitenden Lumen der Bypass-Einheit BE ist die Positionierungs-Einheit PE angeordnet. Die Einheit weist einen katheter-artigen Aufbau auf und umfasst eine Schaftkomponente 9, deren Länge mindestens derart bemessen ist, dass sie vom sogenannten Treitzschen Band, welches den Übergang des Duodenums in das Jejunum markiert, bis in den prä-oralen Bereich bzw. etwa 30 bis 50 cm darüber hinaus reicht. Die Schaftkomponente 9 weist mindestens ein Innenlumen auf, das den Führungsdraht GW aufnimmt, über den hinweg die Vorrichtung bei der Applikation trans-oral und trans-ösophageal in den Magen vorgeschoben, und die im Patienten verweilende Bypass-Einheit vom Magen aus in eine trans-pylorische, durch prä-und post-pylorische Ballonkomponenten fixierte Position gebracht wird. Aus der trans-pylorischen Position der Einheit heraus wird, wiederum dem Führungsdraht folgend, die trans-duodenale Baugruppe TDB bzw. deren auf kurzes, kompaktes Maß geraffter Folienschlauch axial entfaltet bzw. auf seine gesamte Länge axial ausgezogen.

Bei der trans-ösophagealen Passage und trans-pylorischen Fixierung sind sie beiden Einheiten BE und PE, wie in der Figur abgebildet, in spezifischer Weise zueinander angeordnet bzw. positioniert. Die Bypass-Einheit sitzt dem vorderen, distalen Ende der Positionierungs-Einheit auf, wobei die Schaftkomponente der Positionierungs-Einheit durch das zentrale Lumen der Bypass-Einheit verläuft und die distale Spitze der Positionierungs-Einheit einige Zentimeter über das distale Ende der Schaftkomponente der trans-pylorischen Baugruppe hinausreicht. Diese spezifische Position wird durch eine von extrakorporal bedienbare Kopplungs-Einheit KE gesichert. Die koppelnde Einheit besteht vorzugsweise aus einer Ballonkomponente mit nur geringer Dehnbarkeit bzw. hoher Formstabilität. Sie kann mit entsprechend hohem, für eine kraftvolle Koppelung erforderlichen Druck beaufschlagt werden, wobei die bei der Herstellung ausgeformten Abmessungen des Ballons im beaufschlagten Zustand nur geringfügig überschritten werden. Die zuverlässige Koppelung der beiden Einheiten widersteht so den bei der ösophagealen und pylorischen Passage entstehenden, zum Teil nicht unerheblichen, reibungs- und dehnungsbedingten Widerständen. Die Schaftkomponente der Positionierungs-Einheit weist eine entsprechende, für den manuellen, extrakorporal geführten Vorschub geeignete Steifigkeit bzw. Knicksicherheit auf. Die Knickstabilität kann für die trans-ösophageale und/oder trans-pylorische Passage durch eine optionale, zusätzliche in das Lumen der Schaftkomponente einschiebbare, rohr- oder schlauchartige Stabilisierungs-Komponente erhöht werden. Diese kann nach der erfolgten trans-pylorischen Fixierung des gekoppelten Verbundes der Bypass-Einheit mit der Positionierungs-Einheit ganz oder teilweise entfernt bzw. zurückgezogen werden, wodurch sich im Falle eines weiteren Vorschubs der Positionierungs-Einheit in das Duodenum hinein, eine widerstands-reduzierte Biegung bzw. traumareduzierende Schmiegung der Schaftkomponente an den C-förmigen, schlingenartigen Verlauf des Duodenums ermöglicht wird.

Die Schlauchfolie der trans-duodenalen Baugruppe TDG ist im anwendungsfertigen Zustand bzw. bei der ösophagealen und pylorischen Passage in einem kompakt gerafften, paketartigen Zustand auf dem, über die trans-pylorische Baugruppe hinausreichende Schaftanteil der Positionierungs-Einheit aufgefädelt. Die distale Spitze der Positionierungs-Einheit nimmt dabei die henkelartige Schleife 8 der Bypass-Schlauchfolie auf. Die Sicherung der Schleife auf der distalen Spitze der Einheit PE erfolgt durch den an der Spitze austretenden Führungsdraht, der durch eine lochartige Perforation am Apex der Schleife verläuft.

Im Anschluss an die trans-pylorische Fixierung der Vorrichtung bzw. des gekoppelten Verbundes der Bypass- und der Positionierungs-Einheit durch Befüllung der Ballonkomponenten der trans-pylorischen Baugruppe TPG, bieten sich dem Anwender im Rahmen der vorliegenden Erfindung mehrere Optionen für die axiale Entfaltung des folienartigen Bypass-Schlauches:
- die axiale Entfaltung des Folienschlauches kann durch den duodenalen Vorschub der Positionierungs-Einheit PE erfolgen, wobei die folienartige Schlauchkomponente 7 der Schaftspitze der Einheit PE aufsitzt und bei deren Vorschub in das Duodenum mitgenommen bzw. transportiert wird. Um den Vorschub der Einheit PE bzw. deren freie relative Beweglichkeit zu ermöglichen, wird die Kopplung der beiden Einheiten durch Entleerung der Ballonkomponente der Kopplungs-Einheit aufgehoben.
- die Entfaltung des Folienschlauchs kann ferner erfolgen, indem der gekoppelte Zustand der Bypass- und der Positionierungs-Einheit nach erfolgter trans-pylorischer Fixierung der Vorrichtung aufrechterhalten bleibt, und der duodenale Vorschub der Bypass-Schlauchfolie durch einen innerhalb des Schafts der Positionierungs-Einheit PE geführten, dem Führungsdraht GW folgenden, kleinlumigen Katheter mit einer widerstandsgebenden Auflagefläche für die henkelartige Schleife 8, die an der Folienschlauchlomponente 7 endständig angebracht ist. Die axiale Entfaltung der Bypass-Folie kann so, bedingt durch die, der duodenalen Passage angepassten Biege- und Schmiegeeigenschaften der entsprechend kleinlumigen Katheter, insbesondere für ungeübte Anwender einen Anwendungsvorteil bieten.

Ist widerstandsbedingt bzw. wegen eines anzunehmenden Verletzungsrisikos nur eine unvollständige axiale Entfaltung des trans-duodenalen Folienschlausches möglich, kann der noch verbleibende, nicht entfaltete Anteil der Schlauchfolie im Rahmen der Erfindung durch eine in das Binnenlumen der Schlauchfolie eine spülende Flüssigkeit eingeleitet werden, die die Folie axial "ausgerollt" bzw. axial vollständig entwickelt. Die applizierte, im Folienschlauch zum Dünndarm hin abfließende, entsprechend großzügig bemessene Spülflüssigkeit nimmt den Folienschlauch mit sich. Ein Rückschlag der Folie in Richtung des Magens oder eine Verschlingung des Folienschlauchs kann im Rahmen der Spülung durch die Führung des im Lumen des Folienschlauchs verlaufenden Drahtes vermieden werden.

Die Zuleitung der Spülflüssigkeit erfolgt durch eine oder mehrere Öffnungen 14 in ein Inneren der Schaftkomponente 9 der Positionierungs-Einheit geführtes Lumen. Die zuleitenden Öffnungen sind unmittelbar unterhalb bzw. distal der trans-pylorischen Baugruppe TPG angeordnet sind, um die dort, in geraffter Weise verstauten Folienanteile mit dem auf gleicher Höhe erzeugten Flüssigkeitsstrom zu erfassen.

Um bei einer derartigen schlauchentfaltenden Spülung einen magenwärts gerichteten Abfluss von Flüssigkeit zu vermeiden, kann der koppelnde Ballon 11 der Einheit KE während der Spülung für eine passagere magenwärts gerichtete Dichtung des Bypass-Lumens eingesetzt werden.

Um den größtmöglichen Anwendungskomfort und das kleinstmögliche Verletzungsrisiko bei der Applikation der Vorrichtung zu gewährleisten, werden die axialen Biege- und Knickeigenschaften der katheter-artigen Schaftkomponente 9 bzw. der Schaftanteile der Positionierungs-Einheit derart eingestellt sein, dass der Katheterschaft im Verbund mit dem in seinem, im Schaftlumen geführten Draht zum einen, einen von extrakorporal führbaren, knickstabilen Vorschub der gekoppelten Vorrichtungsanteile BE und PE durch die Speiseröhre, den Magen und den pylorischen Schließmuskel ermöglicht, zum anderen aber auch eine möglichst flexibel geschmeidige, reibungsarme Passage des entkoppelten Schaftes der Positionierungs-Einheit oder deren Schaftanteile durch das relativ eng, c-förmig geschlungene Duodenum hindurch gestattet. Um entsprechende Biegeeigenschaften des Schaftes der Positionierungs-Einheit beim trans-ösophagealen und trans-pylorischen Vorschub zu gewährleisten, werden bevorzugt Führungsdrähte größeren Durchmessers und höherer axialer Steifigkeit bzw. Knickstabilität verwendet. In der klinischen Praxis sind bei Gastroskopen Durchmesser von Arbeitskanälen von bis zu 3,8 mm gängig, wobei in diesem Falle im Rahmen der Anwendung Drähte im Durchmesserbereich bis zu 3 mm zur Anwendung kommen.

Alternativ zu höher-kalibrigen Drähten kann auch ein Verbund aus einem kleinkalibrigen Führungsdraht mit beispielsweise 1mm Durchmesser und einer ca. 3 mm Außendurchmesser messenden, die Steifigkeit des Verbundes einstellenden bzw. modifizierenden Kathetereinheit für die schienende Führung der Vorrichtung bzw. ihrer Segmente verwendet werden. Eine derartige Anordnung von Führungsdraht und Katheter kann zeitsparend, direkt durch den Arbeitskanal des Endoskopes geführt werden.

Alternativ hierzu kann über einen initial endoskopisch eingelegten, kleinlumigen Führungsdraht, in einem der Endoskopie nachfolgenden Schritt, eine die Biegeeigenschaften der schienenden Komponente modifizierende Kathetereinheit über den Draht geschoben werden, wobei in diesem Falle Außendurchmesser möglich sind, die gängige Arbeitskanaldurchmesser überschreiten und zusätzliche, in die modifizierende Kathetereinheit integrierte Lumina erlauben.

Die Erfindung sieht ebenfalls segmentiert aufgebaute, schienend wirkende "Führungen" für die Applikation der Vorrichtung vor, wobei das proximale, die ösophageale und pylorische Passage bahnende Segment aus eine höhere Steifigkeit als das distale Segment aufweist, dessen Steifigkeit bzw. Biegefähigkeit für die duodenale Passage angepasst ist. Die beiden Segmente der Kathetereinheit sind axial miteinander fest verbunden und werden in ihrem Innenlumen durch einen kleinlumigen Führungsdraht stabilisiert.

Optional verfügen die biege- bzw. stabilitätmodifizierenden Kathetereinheiten am distalen Ende über eine von außen befüllbare Ballonkomponente, die im befüllten Zustand den Verbund von Führungsdraht und schienendem Katheter innerhalb des Jejunums bzw. in seiner jeweiligen distalen Position vor einer Dislokation sichern können.

Das proximale Segment der Schaftkomponente 9, welches sich vom Pylorus PYL bis nach extrakorporal erstreckt, weist eine Länge von ca. 1,3 bis 1,5 m auf. In der dargestellten Bauform gehen sämtliche Schaftanteile der Positionierungs-Einheit aus einer einzigen, durchgehenden Schaftschlauch-Komponente hervor. Alternativ hierzu sind Bauweisen des Schaftkomponente 9 optional, bei denen der Schaftschlauch aus zusammengefügten Segmenten verschiedener Materialhärte, verschiedener Materialtypen, Durchmesser und Wandungsstärken besteht.

Der im Durchmesser gemessene Spaltraum, der sich zwischen dem Führungsdraht GW und dem, den Draht aufnehmenden Innenlumen der Schaftkomponente 9 ausbildet, sollte 1,5 mm nicht unterschreiten und 4,0 mm nicht überschreiten, um zum einen entstehende Reibungswiderstände zu begrenzen, und zum anderen knickende Stauchungen des Drahtes innerhalb des Lumens der Schaftkomponente zu vermeiden.

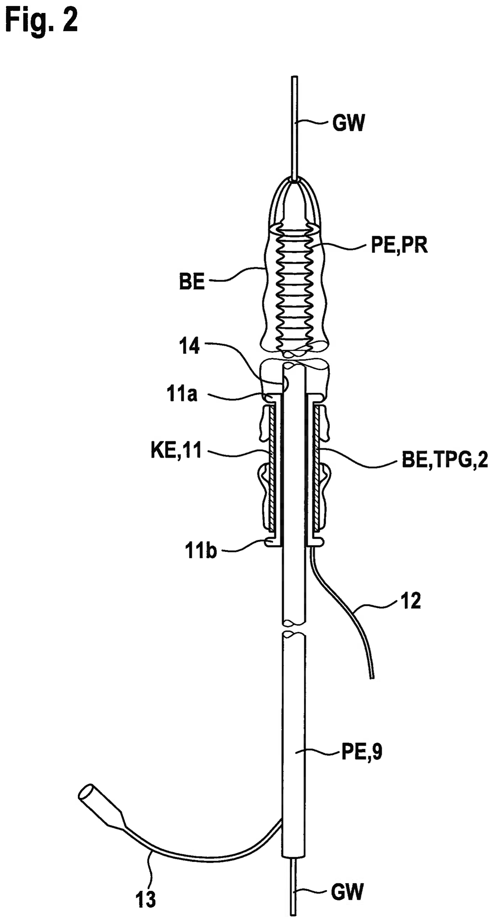

Fig. 2 zeigt eine Ausführungsform der Vorrichtung, bei der die Schaftkomponente der Positionierungs-Einheit PE, im Bereich der Kopplung mit der Bypass-Einheit über das distale Ende der trans-pylorischen Baugruppe TPG hinausreicht, und in überstehenden Bereich eine wellige bzw. korrugierte Profilgebung PR aufweist. Der korrugiert ausgeführte Schaftanteil verleiht dem distalen Ende der Einheit PE bei der Passage der duodenalen Windungen eine vorteilhaft spannungsreduzierte, axiale Biegsamkeit bis zu ca. 180 Grad, und erleichtert so den duodenalen Vorschub. Durch die erreichte Reduktion der elastischen Rückstellwirkungen im vorderen Schaftbereich können zudem bei der Retraktion des Schaftes aus der entfalteten Bypass-Schlauchfolie ungewünschte Reibungswirkungen und damit einhergehende Mitnahmewirkungen auf die Schlauchfolie reduziert werden.

Die wellige Profilgebung bzw. Korrugation PR des distalen Schaftanteils kann beispielsweise durch einen Blasformungsprozess in die Schaftwandung eingeprägt werden, wobei der jeweilige Schaftanteil erhitzt, mit Druck beaufschlagt, im heißen Zustand umgeformt und anschließend durch Kühlung in seiner wellig geprägten Form fixiert wird. Neben einer Umformung das distalen, über die Baugruppe TPG hinausreichenden Schaftanteils, kann die Korrugation des Katheterschaftes auch auf den gesamten intra-korporal applizierten Schaftkörper angewendet werden oder auch auf bestimmte, ausgesuchte Bereiche beschränkt sein, die für eine spannungsreduzierte Passage der Positionierungs-Einheit vorteilhaft sind.

Um die Option der mechanischen, dislokations-sichernden Kopplung bzw. lumenverschließenden Dichtung der Positionierungs-Einheit PE innerhalb der trans-pylorischen Baugruppe TPG der Bypass-Einheit BE, unabhängig von der jeweiligen relativen Position der Einheiten zu erhalten, ist die Kopplungs-Einheit KE bei dieser Bauform nicht fest mit der Oberfläche des Schaftkörpers 9 der Einheit PE verbunden. Statt mit beiden Ballonenden auf der Oberfläche des Schaftes aufgeklebt oder verschweißt zu sein, wird der koppelnde Ballon als ein, in sich geschlossener Hohlzylinder 11 hergestellt, durch dessen inneren Hohlraum bzw. zentralen Kanal der Schaft der Positionierungs-Einheit geführt werden kann. Der Schaft kann somit innerhalb der Baugruppe TPG vom Anwender mechanisch fixiert werden. Ferner kann das Restlumen des Kanals der Baugruppe TPG lumenverschließend gedichtet werden.

Um bei Verschiebungen des Schaftes 9 innerhalb der Baugruppe TPG eine Dislokation des Kopplungs-Einheit KE zu vermeiden, wird der hohlzylindrische Ballonkorpus 11 bei der Ausformung mit zwei endständigen, schulter-artigen, als Anschlag dienenden Erweiterungen 11a und 11b ausgestattet, Die Bauform der Kopplungs-Einheit erfordert es, dass der koppelnde Hohlzylinder über eine separate, nicht in die Schaftwandung der Positionierungs-Einheit integrierte, freie Schlauchzuleitung 12 befüllbar ist.

Die Wandung des Schaftkörpers 9 weist ein optionales Lumen 13 auf, welches für die Zuführung von Luft oder von haftungs- und reibungsreduzierenden Substanzen in den Binnenraum der Bypass-Schlauchfolie 7 verwendet werden kann. Das Lumen mündet über eine Öffnung 14 in den Binnenraum der trans-duodenalen Folienschlauch-Baugruppe.

Die Figur zeigt ferner die Ballonkomponenten 3,4 und 5 im nicht befüllten Zustand auf dem Schaftelement der Baugruppe TPG.

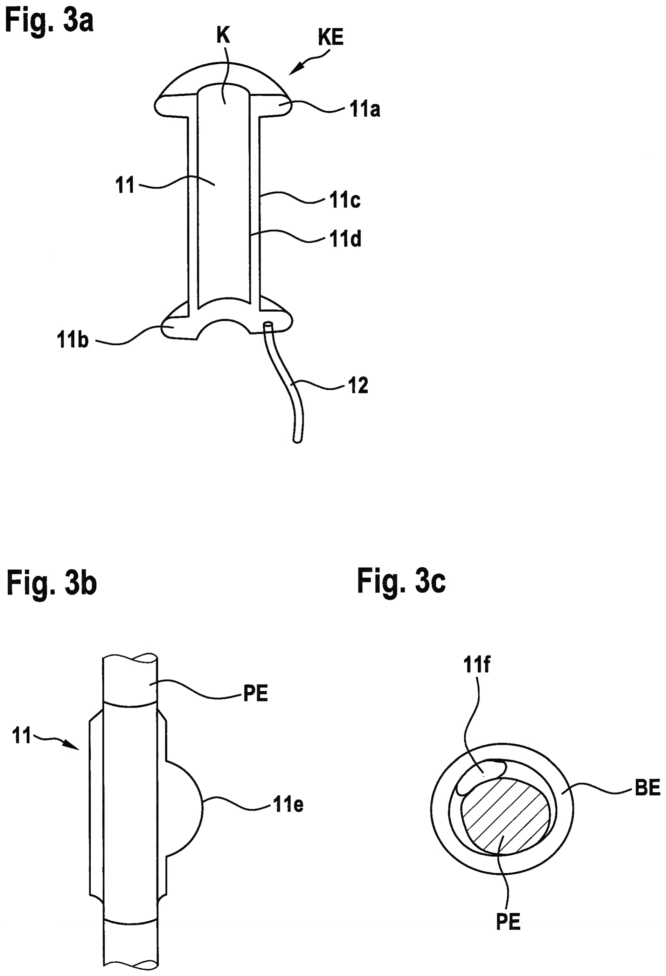

Fig. 3a zeigt eine Bauform einer Kopplungs-Einheit KE, basierend auf einem als Hohlzylinder ausgeführten Ballonkorpus, der im Zentrum einen durchgängigen Kanal K ausformt, der die jeweilige Positionierungs-Einheit PE in sich aufnimmt. Der Hohlzylinder 11 wird im Spaltraum zwischen der Oberfläche der Positionierungs-Einheit PE und der Oberfläche des durchleitenden Kanals der trans-pylorischen Baugruppe TPG positioniert.

Der Hohlzylinder 11 der Kopplungs-Einrichtung KE ist weder mit der Bypass-Einheit BE noch mit der Positionier-Einheit PE baulich fest verbunden. Die für das freie gegenseitige Verschieben der Einheiten KE, BE, PE erforderliche, zu erhaltende Position der Kopplungs-Einrichtung KE zwischen den Einheiten BE und PE wird durch besondere, in den Ballonkorpus 11 geformte, wulst- bzw. kragenartige Ausformungen 11a und 11b im Bereich der Stirnseiten des Hohlzylinders 11 unterstützt.

Die Befüllung der Kopplungseinheit erfolgt bevorzugt mit einem flüssigen Medium. Die Zuleitung von extrakorporal erfolgt durch einen separate Schlauchleitung 12.

Im mit Druck beaufschlagten Zustand stemmen sich die äußere und die inneren Wandungen 11c und 11d des Hohlzylinders 11 gegen den Schaftkörper 9 der Positionier-Einheit PE einerseits sowie gegen die Innenfläche des durchleitenden Kanals in dem Schaftkörper 2 der Bypass-Einheit BE andererseits. Die "flotierende", nicht fest verbundene Ausführung der Kopplungs-Einrichtung KE gewährleistet, dass nach erfolgter Platzierung der Baugruppe TPG im Pylorus PYL ein frei gerichteter Schub bzw. Versatz der Positionier-Einheit PE innerhalb der Bypass-Einheit BE möglich ist, wobei die Kopplungs-Einrichtung KE beim Vorschub oder beim Rückzug der Positionier-Einheit PE nicht in das Duodenum DU oder in den Magen ST mitgenommen wird und so ihre koppelnde und/oder dichtend verschließende Funktion innerhalb der trans-pylorischen Baugruppe TPG erfüllen kann.

Der Ballonkörper der Kopplungs-Einheit besteht vorzugsweise aus einem Material mit geringer Volumendehnbarkeit (Compliance), beispielsweise aus einem thermoplastischen Polyurethan der Härte nach Shore von 90A bis 99A oder von 55D bis 65D. Die Wandung des Hohlzylinders weisen Wandungsstärken von ca. 10 bis 40 µm, bevorzugt 15 bis 30 µm auf.

Fig. 3b zeigt eine Ausführungsform der Kopplungs-Einheit KE, wobei der koppelnde Ballon 11 an seinen beiden Enden mit der Oberfläche der Positionierungs-Einheit baulich fest verbunden ist. Der Ballon kann im mittigen Segment eine sphärische Erweiterung 11e aufweisen, wodurch die befüllte Sphäre auch außerhalb des Kanals der Baugruppe TPG, also innerhalb des Binnenlumens der Bypass-Schlauchfolie für einen dichtenden Verschluss verwendet werden kann, wodurch im Rahmen einer Spülung des Folienlumens oder einer beabsichtigten Beaufschlagung des Folienschlauches mit Druck ein Verschluss in proximaler Position zum jeweils eingeleiteten Medium hergestellt werden kann. Die Befüllung erfolgt über in die jeweiligen Schaftkörper der Einheit PE integrierte Lumina.

Fig. 3c zeigt eine weitere Ausführungsform einer koppelnden Funktion in einem transversalen Querschnitt, wobei die koppelnde Komponente aus einem einfachen, zylindrischen Ballonkörper 11f besteht, der im Spaltraum zwischen den Einheiten PE und BE positioniert ist. Der koppelnde Ballon kann wegen seiner geringen Baugröße auf der Positionier-Vorrichtung PV fest aufgebracht bzw. an seiner zylindrischen Seitenfläche mit dem Schaftkörper der Einheit PE verklebt sein. Er kann optional auch frei beweglich und separierbar verbaut sein. Die Befüllung erfolgt über entsprechende Lumina im Schaftkörper der Positionierungs-Einheit oder durch frei verlaufende Schlauchzuleitungen.

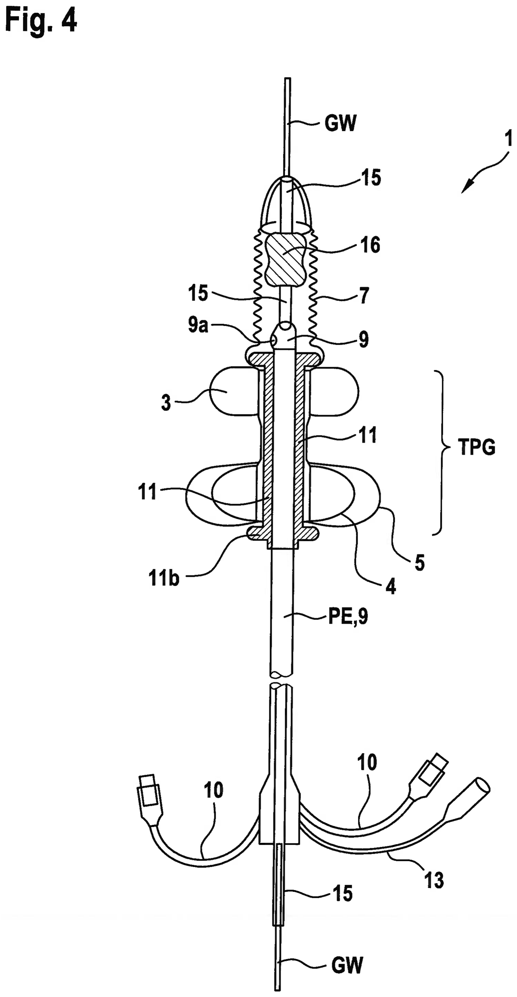

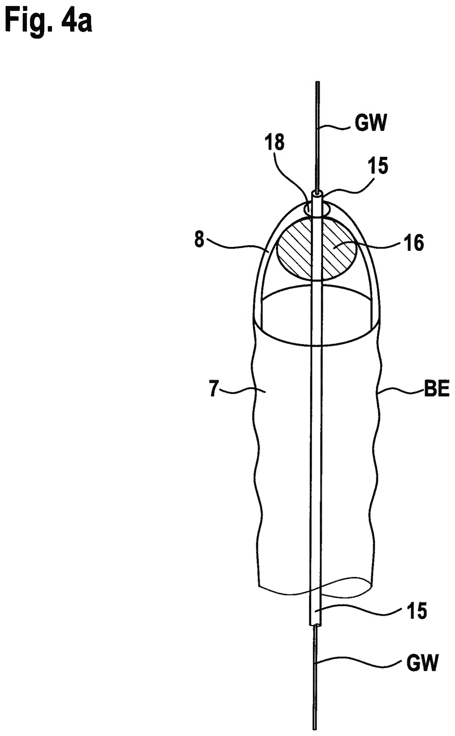

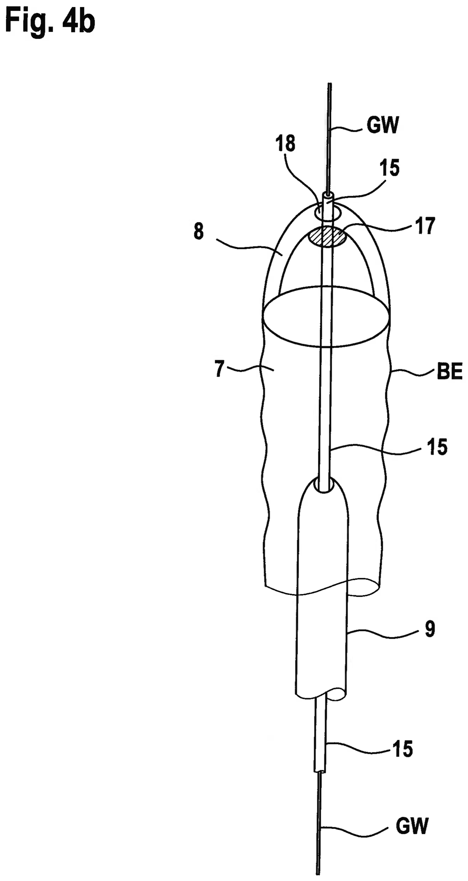

Fig. 4 zeigt eine Ausführungsform der Vorrichtung 1, bei der der relativ rigide, den knickstabilen Vorschub der Vorrichtung durch den Ösophagus und den Pylorus gewährleistende Schaftkörper 9 der Positionierungs-Einheit PE am distalen Ende der trans-pylorischen Baugruppe TPG endet, und die axiale Entfaltung der trans-ösophagealen Schlauchfolie 7 der Baugruppe TDG durch eine separate, innerhalb des Schaftes 9 geführte Kathetereinheit 15 kleineren Durchmessers erfolgt, wobei die separate Kathetereinheit eine relativ zum Schaftkörper 9 erhöhte axiale Biegsamkeit aufweist, wodurch deren duodenale Passage beim Vorschub und auch die Retraktion des Katheters erleichtert wird. Das den Katheter 15 führende Lumen des Schaftkörpers 9 wird dabei derart passgenau bemessen, dass das Lumen den Außendurchmesser des Katheters 15 mit einem Spaltraum von in Summe im Durchmesser etwa 0,2 bis 0,5 mm aufnimmt, um so den freien gegenseitigen Versatz, aber auch einen nach proximal gerichteten, dichtenden Effekt für den Fall einer Beaufschlagung des Binnenraums der Schlauchfolie 7 mit Druck oder einer Spülung des Schlauchfolien-Binnenraums von proximal nach distal, um die axiale Entfaltung der Bypass-Schlauchfolie zu unterstützen.

Der Austritt der Spüllösung bzw. des mit Druck beaufschlagenden Mediums erfolgt am distalen Ende des Schaftkörpers 9 durch eine oder mehrere Öffnungen 9a. Die Zuleitung des Mediums erfolgt über einen extrakorporalen Konnektor 13.

Der proximale, in den Bereich des duodenalen Segmentes der trans-pylorischen Baugruppe reichende Anteil des Schaftkörpers der Positionierungs-Einheit kann beispielsweise aus einem Polyurethan der Härte nach Shore 80A bis 95A hergestellt werden, wobei der äußere Durchmesser ca. 7 bis 10 mm aufweist, bei Wandungsstärken des Schaftkörpers von ca. 1,5 bis 2,5 mm, bevorzugt 1,8 bis 2,2 mm. Auch eine Ausführung des proximalen Schaftkörpers in PVC ist möglich.

Der Schaft der im Inneren des Schaftkörpers 9 geführten katheterartigen Einheit 15 besteht bevorzugt aus einem rigiden Polymer, beispielsweise aus PBAX- oder Polyamid-Typen, die auch im niedrigen Wandstärkenbereich von beispielsweise 0,2 bis 0,3 mm knickstabil schienende Eigenschaften aufweisen und insbesondere ziehharmonikaartige Stauchungen der Katheterwandung beim Vorschub der Kathetereinheit 15 durch das Lumen des Schaftkörpers 9 vermeiden.

Fig. 4a Die Kathetereinheit 15 trägt am vorderen Ende einen sich vorzugsweise elastisch aufdehnenden und retrahierenden, beispielsweise aus isopren-basierten Polymeren mit hoher Volumendehnbarkeit (Compliance) hergestellten Ballon 16, der im befüllten Zustand zum einen den Vorschub der Katheterspitze bzw. deren freien, widerstandsreduzierten Lauf durch das Duodenum bahnt, zum anderen, bei einer entsprechend nach proximal, in das vordere Ende des Folienschlauches hineinreichend verlängerten Bauweise, im vollständig befüllten Zustand das distale Ende der Folienschlauchkomponente 7 lumenverschließend dichten kann.

Die Figur beschreibt ferner die henkelartige Komponente 8. Die beiden Enden der Komponente sind in einer um 180 Grad versetzten Position am distalen Rand des Folienschlauches 7 angebracht. Die schleifenartige Formation ist in ihrer Länge derart bemessen, dass sie über dem distalen Rand der Schlauchfolie einen Scheitelpunkt ausbildet, der im freien, nicht durch Schub verformten Zustand mindestens 30 mm beabstandet ist. Die Komponente besteht aus einem Material, mit geringen elastischen Aufrichtungseigenschaften, um unerwünschte, ankernd wirkende Effekte innerhalb des Darmlumens zu verhindern, wobei sich die Enden der streifenförmig zugeschnittenen Schlaufe in elastischer Weise aufrichten und sich gegen die Wandung des Darms stemmen und so eine freie axiale Entwindung bzw. axiale Ausrichtung des Folienschlauches beeinträchtigen können.

Fig. 4b In einer alternativen Ausführung das Katheters 15 wird auf den endständigen Ballonkörper 16 verzichtet. Die Spitze der Schaftkomponente des Katheters ist hingegen mit einer Formation 17 versehen, der die henkelartige Schleife 8 mit ihrem apikalen Scheitel der der Katheterspitze sattelartig aufliegt, wobei die relative Position der Spitze zur Schleife durch den Führungsdraht GW, der durch eine apikal in der Schleife angebrachte Öffnung 18 hindurchgeführt ist, gesichert wird.

Die beim duodenalen Vorschub des Katheters erforderliche Mitnahme der Schleife 8 wird durch die Auflage der Schleife auf die sattelartige Formation gewährleistet.

Eine entsprechende sattelartige, distal endständige Formation 17 kann auch in Kombination mit einer endständigen Ballonkomponente auf dem Katheterschaft 15 aufgebracht werden. Die entsprechende Ballonkomponente 16 kann dabei unmittelbar proximal zur sattelartigen Formation angeordnet, oder auch soweit proximal von ihr beabstandet sein, dass die Ballonkomponente im Bereich des distalen Schlauchfolienendes der Baugruppe TDG positioniert ist und dort einen passageren dichtenden Verschluss des distalen Bypass-Lumens herstellen kann.

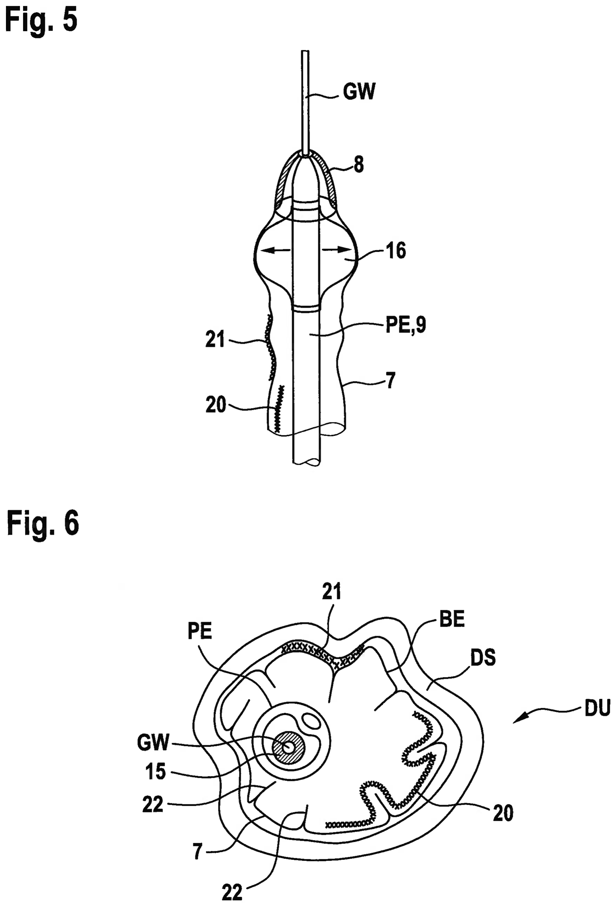

Fig. 5 zeigt das distale Ende einer Positionierungs-Einheit PE im Verbund mit dem distalen Ende der Bypass-Schlauchfolie 7, wobei die Schaftspitze der Einheit PE mit einem lumenverschließenden Ballonelement 16 ausgestattet ist, das von extrakorporal befüllt und entleert werden kann. Die Ballonkomponente besteht hier aus mikro-dünnwandigem Polyurethan-Material, welches im entleerten Zustand derart gering auftragend auf der Schaftoberfläche der Positionierungs-Einheit PE kollabiert werden kann, dass die entleerte Ballonhülle bei der Retraktion des Positionierungs-Einheit PE aus der trans-duodenal entfalteten Bypass-Schlauchfolie 7 einen niedrigen Reibungswiderstand bzw. niedrige Adhäsionswirkungen zur Innenfläche der Schlauchfolie hin entwickelt. Bei der besonders anwendungskritischen Retraktion der Positionierungseinheit PE kann somit die Wahrscheinlichkeit der Lösung der duodenal entfalteten Schlauchfolie 7 von der ihr anliegenden Darmschleimhaut reduziert werden. Widerstands- und adhäsionsbedingte Effekte bei der Retraktion der Einheit PE können ferner reduziert werden, in dem im Moment der Retraktion Luft oder flüssige Medien in den befüllten Binnenraum der Schlauchfolie 7 eingebracht werden, die beispielsweise lubrifizierend wirken und/oder die Oberflächenadhäsion reduzieren bzw. hemmen, und somit zueinander entgegengesetzt gerichtete Gleitbewegungen der Einheit PE einerseits und der Schlauchfolien-Komponente 7 andererseits in einer in Summe widerstandsreduzierenden Weise ermöglichen.

Fig. 6 beschreibt weitere bauliche Optionen und Ausstattungen der Vorrichtung, die die adhäsive Anhaftung der Folienschlauchkomponente 7 an der Darmschleimhaut DS erhöhen, indem die der Darmschleimhaut zugewandte Oberfläche der Schlauchkomponente beispielsweise mit einer wasserbasierten, gelartigen Substanz 21 benetzt ist oder alternativ auch eine fettbasierte, salbenartige Substanz auf ihrer Oberfläche trägt. Es sind ferner trockene zellulose-basierte Beschichtungen der Folienoberfläche denkbar, die unter Aufnahme von Wasser gelartig aufquellen und im benetzten Zustand einen klebend adhäsiven Effekt entwickeln, der über die Adhäsionswirkung eines physiologischen, wässrigen bzw. schleimhautypischen, muzinhaltigen Films hinausgeht.

Während der lumendichtende Verschluss des Binnenraumes der Schlauchfolie im proximalen Bereich durch eine beispielsweise hohlzylindrisich aufgebaute Kopplungs-Einheit KE innerhalb der trans-pylorischen Baugruppe der Bypass-Einheit hergestellt wird, oder durch die Ballonkomponente 11 innerhalb des Duodenums erfolgt, wird der Lumenverschluss im distalen Bereich der Schlauchfolie durch Ballonelemente 16, die entweder der Schaftkomponente der Positionierungs-Einheit oder einer separaten, durch den Katheterschaft der Einheit PE geführten Ballonkatheter 15 hergestellt. Die erforderliche relative Position zwischen dem lumenverschließenden Ballon und dem distalen Enden der Schlauchfolie 7 wird durch den aboral gerichteten Vorschub der Positionierungs-Einheit gewährleistet, wobei die Spitze der jeweiligen, vorschiebenden, katheterartigen Komponente die schleifenartige Formation 8 aufnimmt bzw. unter eine nach aboral gerichtete Zugspannung nimmt. Die distale Kante der Schlauchfolie kann somit beispielsweise 4 bis 5 cm von der distalen Schulter des Ballons 16 beabstandet sein, wodurch der befüllte Ballon eine ausreichende Auflagefläche entwickelt und das Herausgleiten aus der Mündung der Schlauchfolie verhindert werden kann.

Wird der so abgeschlossene Binnenraum der Schlauchfolie mit Druck beaufschlagt, entfaltet sich der Folienquerschnitt vollständig und liegt der Schleimhaut mit größtmöglicher Fläche an. Wird der Druck entlastet, stellt die radial wirkende Kraft des Dünndarms den Darm auf einen kleineren Querschnitt bzw. Durchmesser zurück, wodurch sich Wandanteile des Folienschlauches im Darmlumen in eine "residuale" Faltung 22 legen, ohne der Darmschleimhaut exponiert zu sein. Die initiale vollständige Entfaltung und nachfolgende Einfaltung der Folienschlauchhülle in radial eingestülpte "Reservefaltung" maximiert die mögliche Adhäsionsfläche zwischen Folie und Darm. Sie hält die Schlauchfolie in ihrer trans-duodenal durchleitend wirkenden Position bzw. gewährleistet die für die ungehinderte Passage von Mageninhalt erforderliche axiale, nicht torquierte Ausrichtung der Schlauchfolie.

Die Figur beschreibt schematisch das Zusammenspiel von Adhäsions- und Reibungseffekten, die zwischen der Darmschleimhaut DS, der Folienschlauchkomponente der trans-duodenalen Baugruppe und den Komponenten der Positionierungs-Einheit PV wirken. Die sich zueinander relativ bewegenden bzw. verschobenen Komponenten werden in einem schematischen, transversalen Querschnitt durch das Lumen des Duodenums DU dargestellt. Im mit Luft befüllten bzw. mit einem moderaten Luftdruck beaufschlagten Zustand liegt die Oberfläche der Schlauchfolie 7 der mit natürlichem Sekret benetzten Darmschleimhaut DS flächig an. Die adhäsiv wirkende Verbindung der Schlauchfolie zur Darmschleimhaut kann durch adhäsiv wirkende Pasten, Gele oder gelartig quellende Substanzen 21 zwischen der Schlauchfolie und der Darmschleimhaut gesteigert werden. Im Inneren der Schlauchfolie verläuft der distale Schaftanteil der Positionierungs-Einheit der Vorrichtung. Die Figur zeigt ferner eine oberflächenwirksame Substanz 20, die adhäsive und reibend wirkende Effekte zwischen der Innenfläche der Schlauchfolie und der Oberfläche der im Inneren des Folienschlauchs liegenden bzw. bewegten Komponenten der Einheit PE reduziert. Es kommen beispielsweise seifenartige, ölbasierte oder silikonöl-basierte Substanzen zur Anwendung.

Der äußere Durchmesser des Schaftkörpers der Positionierungs-Einheit PE kann bspw. etwa 5 mm betragen, also etwa 1/4 bis 1/5 des Durchmessers der trans-duodenal applizierten Schlauchfolie 7, die im vollständig radial entfalteten Zustand einen Durchmesser von etwa 20 bis 30 mm aufweist. Der Umfang der Schlauchfolie liegt in diesem Falle rechnerisch bei etwa 63 bis etwa 94 mm. Der Umfang des Katheters liegt entsprechend bei etwa 15 mm. Das Verhältnis der beiden Umfangsmaße liegt bei ca. 4,2 zu 1 bzw. bei ca. 6,3 zu 1. Für ein vorteilhaftes, die Ablösung des Folienschlauchs von der Darmschleimhaut vermeidendes Kräfteverhältnis zwischen Adhäsion und Reibungswirkung schlägt die Erfindung bei intra- oder trans-duodenal ein- oder durchgeführten Schaftkörpern mit Außendurchmessern von ca. 5 mm ein Umfangsverhältnis von 4 zu 1 bis 6 zu 1 vor, das nicht unterschritten werden sollte.

Die Erfindung bevorzugt die Kombination der beschriebenen Umfangverhältnisse mit einer gleichzeitigen Reduktion der Adhäsions- und Reibungseffekte durch entsprechende Substanzen, die in den Binnenraum der Schlauchfolie eingebracht werden, sowie die zusätzliche Steigerung des adhäsiven Effektes des Folienschlauches an der Darmschleimhaut durch beispielsweise gel- oder salbenartige Substanzen, um die Effizienz bzw. Stabilität der trans-duodenalen Positionierung der Schlauchfolie zu verbessern, und so Ablösungen der im Darmlumen entfalteten Schlauchfolie bei der Retraktion der Positionierungs-Einheit aus der im Patienten verweilenden Bypass-Einheit der Vorrichtung zu vermeiden.

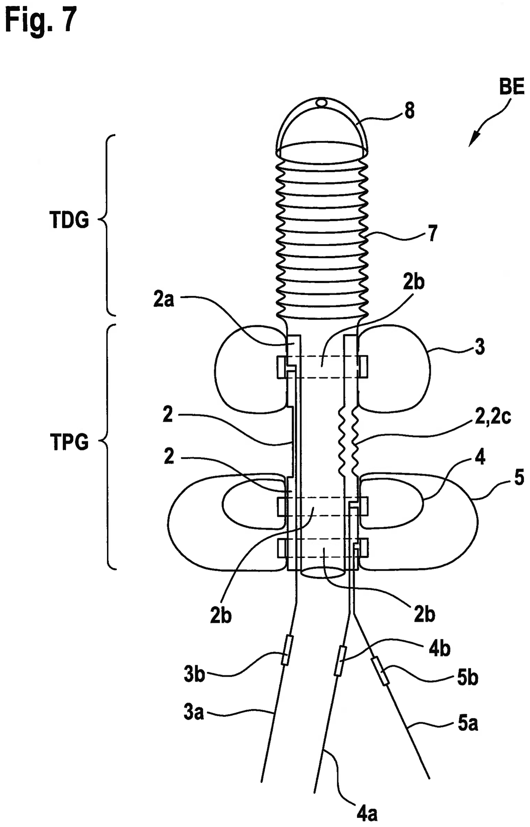

Fig. 7 beschreibt bauliche Details der im Patienten verweilenden Bypass-Einheit BE. Die Einheit umfasst eine trans-pylorische Baugruppe TPG, die mit mehreren Ballonkomponenten 3, 4 und 5 ausgestattet ist, die die Bypass-Einheit in ihrer trans-pylorischen Lage sichern. Die Ballonkomponenten sitzen einer schlauch- bzw. rohrartigen Schaftkomponente 2 auf. An das distale Ende 2a der Schaftkomponente schließt sich eine im wesentlichen folienbasierte trans-duodenale Baugruppe TDG an. Die Einheit umfasst eine ca. 60 cm langes Folienschlauchkomponente 7, an deren distalen Ende eine henkelartige, als Folie bestehenden Formation 8 angebracht ist.

Die Bypass-Einheit BE weist im post-pylorisch positionierten Segment der trans-pylorischen Baugruppe einen sphärisch oder diskoid ausgeformten Ballon 3 auf, der sich im befüllten Zustand in den sogenannten duodenalen Bulbus hineinentwickelt und dort eine Widerlagerfunktion zum pylorischen Schließmuskel gewährleistet, die eine magenwärts gerichtete Dislokation der Bypass-Einheit ausschließt. Im Bereich des prä-pylorisch positionierten, magenseitigen Segmentes der Baugruppe trägt die Schaftkomponente der Durchleitungs-Einheit zwei, zueinander konzentrisch angeordnete Ballonkomponenten, wobei die äußere Ballonkomponente 5 die innere Komponente 4 vollständig einhaust. Die innere Komponente dient als sicherheitsredundantes Widerlager, für den Fall, dass es im langfristigen Platzierungsverlauf zu einer partiellen oder völligen Entleerung der äußeren Ballonkomponente kommt. Der vorzugsweise mit einer Flüssigkeit befüllte, diskoid ausgeformte Ballon 4 verhindert das Abgleiten der Durchleitungs-Einheit in das Duodenum und Jejunum und beugt so einem Darmverschluss vor. Der Ballon wird daher vorzugsweise mit einer eigenen, unabhängigen Befüllzuleitung 4a ausgestattet. Der äußere Ballon 5 wird über die Zuleitung 5a befüllt, der duodenale Ballon 3 über die Zuleitung 3a. Alternativ können die Ballons 3 und 5 auch durch eine gemeinsame Kanal- und Schlauchzuleitung mit einem gasförmigen oder flüssigen Medium befüllt werden.

Die Erfindung schlägt ferne eine besondere, vereinfachte Bauweise vor, bei der der duodenale Ballon und der innere gastrische Ballon über jeweils separate Leitungen befüllt werden, und der äußere gastrische Ballon nur passiv dem inneren Ballon folgt. Er dient lediglich als schützende Hülle bzw. als Barriere, die den direkten Kontakt des ankernd wirkenden inneren Ballons mit den aciden, hydrolytisch wirkenden Sekreten des Magens. Die Hülle des äußeren Ballons ist bevorzugt mit einer zweilagigen Wandung ausgestattet, deren äußere Lage polyamid- bzw. pebax-basiert ist. Sollte es zu einem Versagen des äußeren Ballons kommen, tritt ein dort vorrätiger Farbstoff aus und wird im Urin vom Patienten erkannt. Versagt der innere Ballon tritt das Füllvolumen in den äußeren Ballon über, wodurch die Ankereigenschaften der gastrischen Ballonanordnung erhalten bleiben.

Um dem Patienten das strukturelle Versagen bzw. die Verletzung eines der flüssig- oder gasbefüllten Ballons anzuzeigen, kann im jeweiligen Ballon ein nierengängiger Farbstoff in flüssiger oder trockener Form eingebracht sein, der beim Austritt aus der Ballonhülle vom Darm aufgenommen wird und schließlich vom Patienten im Urin erkannt werden kann.

Die jeweiligen Zuleitungen zu den Ballons werden durch den Ösophagus nach außen geleitet. Die Befüllung mit dem jeweiligen Medium erfolgt bevorzugt volumendefiniert, durch konventionelle Spritzen. Das Volumen wird vorzugsweise derart gewählt, dass die Ballonkomponenten, die allseitigen, direkten Kontakt mit anliegenden Geweben haben, wie insbesondere im Bereich des Bulbus duodeni, in einem partiell befüllten, spannungslos schlaffen Zustand der Ballonhülle verbleiben. Das jeweilige zugeführte Volumen sollte etwa 80 bis 90% des jeweils vollständig, spannungslos bzw. ohne Dehnung der Ballonhülle entfalteten, auf dem Schaftkörper montierten Ballons betragen.

Das distale Segment der zuleitenden Schläuche 3a, 4a und 5a kann im Übergangsbereich 3b, 4b und 5b zur Schaftkomponente der trans-pylorischen Baugruppe derart präformiert, beispielsweise in seiner Wandstärke derart reduziert sein, dass es durch ein scheren- oder zangenartiges Werkzeug, welches über den Arbeitskanal des Endoskopes in den Magen eingeführt und von außen manipuliert wird, durchtrennt und somit von der im Körper verbleibenden Bypass-Einheit entkoppelt werden kann. Die Ballonkomponenten der Bypass-Einheit werden über besonders niederkomplex gestaltete, selbstdichtend wirkende Ventilkomponenten befüllt. In den bevorzugten Ausführungsformen wird der selbstdichtende Verschluss durch flache, gummi-artig elastische Ringkomponenten hergestellt, die den jeweiligen, zuleitenden Öffnungen im ballontragenden Schaft unter elastischer Spannung, zu den Seiten hin flächig überlappend, in dichtender Weise aufliegen. Die dichtenden Ringkomponenten 2b bestehen vorzugsweise aus Materialien mit dauerhaften, nicht ermüdenden elastischen Dehnungseigenschaften, wie diese beispielsweise von Polyurethan oder Silikon gewährleistet werden. Die ventiltragenden Schaftabschnitte weisen optional jeweils zwei zirkuläre, laterale Erhöhungen auf, die den dichtenden Ring in einbettender Weise zwischen sich sichernd aufnehmen.

Das mittige, zwischen den duodenalen und gastrischen Ballons liegende, rohr- oder schlauchartige Schaftsegment 2 nimmt den pylorischen Schließmuskel PYL auf. Die Schaftwandung kann optional in einer wellig korrugierten Weise 2c ausgeformt sein, um die axialen Biegeeigenschaften des pylorisch durchleitenden Segmentes zu verbessern und ein lumenverschließendes Knicken des Schaftes zu vermeiden.

Die Folienschlauchkomponente 7 der Bypass-Einheit besteht aus säurestabilem, hydrolysefestem Material, in vorzugsweise niedrigen Wandstärken im Bereich von 10 bis 40 µm, bevorzugt 15 bis 25 µm. Insbesondere werden TPU-basierte Materialien höherer Shorehärte eingesetzt werden, beispielsweise im Härtebereich von 90A bis 95A und 55D bis 65D. Alternativ können Teflon-basierte oder auch Polyolefinbasierte Polymere verwendet werden. Der frei entfaltete Durchmesser der Schlauchkomponente liegt zwischen 20 und 45 mm, bevorzugt 25 und 35 mm. Die Schlauchfolie kann wellig korrugiert ausgeformt werden, was zum einen die Neigung axialer Verwindung bzw. Torsion vermindert, zum anderen die kompakte Raffung bzw. Faltung der Folie auf der distalen Spitze der Schaftkomponente der Positionierungs-Einheit erleichtert.

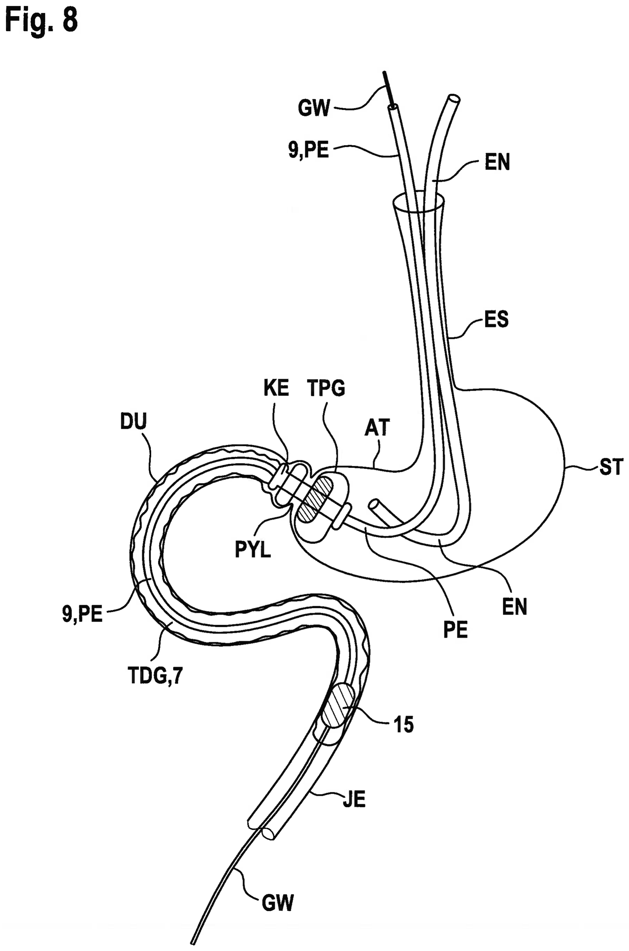

Fig. 8 beschreibt die Applikation bzw. das Einbringen der erfindungsgemäßen, trans-duodenalen Bypass-Vorrichtung in den Verdauungstrakt des Patienten, wobei insbesondere die relative Position der verschiedenen Einheiten der Vorrichtung zu den ihnen benachbarten anatomischen Strukturen veranschaulicht wird. Die Figur beschreibt ferner das funktionelle Zusammenspiel der im Patienten verbleibenden Bypass-Einheit BE mit einer beispielhaften, im Anschluss an die vollständige Einbringung bzw. Entfaltung der Bypass-Einheit entfernten Positionierungs-Einheit PE, sowie eines im Magen ST positionierten, die Applikation beobachtenden bzw. unterstützenden Endoskopes EN.

Das Endoskop wird in etwa parallel zum Schaft der Positionierungs-Einheit PE im Magen positioniert. Die Optik des Endoskopes wird im Magen derart ausgerichtet, dass sie den Magenausgangsbereich (Antrum) AT des Magens sowie den magenseitigen Schließmuskel (Pylorus) PYL darzustellen vermag. Die direkte Sicht ermöglicht es dem Anwender, während der verschiedenen Phasen der Applikation bzw. Einbringung und Manipulation der Vorrichtung die trans-pylorische Lage der trans-pylorischen Baugruppe TPG zu bestätigen bzw. Dislokationen auszuschließen. Über den Arbeitskanal des Endoskopes kann der Magen zudem mit Luft befüllt werden, um die Sichtung des magenseitigen Pylorus zu verbessern.

Die Figur zeigt eine vollständig durch das Duodenum DU hindurch, in das sich anschließende Jejunum JE reichende, axial entfaltete Schlauchfolie 7 der trans-duodenalen Baugruppe TDG. Die Figur zeigt ferner eine optionale, innerhalb der Schlauchfolie positionierte Ballonkomponente 15, die im befüllten Zustand eine lumenverschließende Dichtung des distalen Schlauchfolienendes ermöglicht und im Verbund mit einer proximalen Dichtung der Schlauchfolie im Bereich der trans-pylorischen Baugruppe, eine stauend wirkende, druckbeaufschlagte Befüllung des Binnenraums der Schlauchfolie ermöglicht, wodurch sich die Schlauchfolie radial entfaltet, den ihr exponierten Darm dabei in Dehnung nimmt, und nach erfolgter Entlastung des Binnenraums in einen der Darmschleimhaut allseits angeschmiegten, radiale Einstülpungen der Schlauchhülle ausbildenden, dem jeweiligen Lumen des Darms angepassten, spannungslosen Zustand übergeht.

Ist die Schlauchfolie vollständig in das duodenale bzw. das jejunale Darmlumen entwickelt, werden die Komponenten der Positionierungs-Einheit aus dem Jejunum, dem Duodenum, dem Magen und Osöphagus durch Zugwirkung von extrakorporal entfernt.

Im Anschluss an die Entfernung der Positionierungs-Einheit PE wird über den Arbeitskanal des noch im Magen verweilenden Endoskops eine scheren- oder zangenartige Vorrichtung in den Magen eingeführt, mit der die Befüllleitungen der Ballons der trans-pylorischen Baugruppe in unmittelbarer Nähe der Baugruppe durchtrennt werden. Die Befüllleitungen werden dann, ebenso wie das Endoskop, aus dem Patienten entfernt.

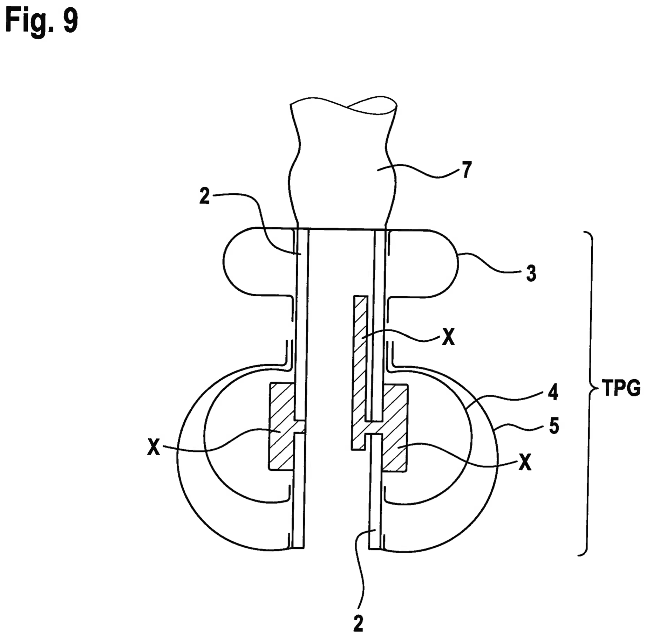

Fig. 9 beschreibt eine besonders fortgeschrittene Bauweise der Bypass-Vorrichtung, die eine kontinuierlich volumetrisch messende Funktion als wesentliche Funktion integriert. Erfasst wird die Menge Mageninhalt, die über den Pylorus des Magens pro Zeiteinheit in das sich dem Magen anschließende Duodenum abfließt. Die besondere Ausstattung der Einheit umfasst ferner die Option einer qualitativen und ggf. auch quantitativen oder semi-quantitativen Erfassung des jeweiligen Gehaltes an Fetten, Proteinen und/oder Kohlenhydraten.

Für die fortlaufende volumetrische Messung kann ultraschallbasierte Sensortechnik verwendet werden. Entsprechende Sensoren sind im Stand der Technik bekannt und werden beispielsweise bei der volumetrischen Messung von Urin angewendet.

Bei der qualitativen bzw. kombiniert qualitativen/quantitativen Bestimmung von spezifischen Molekülen oder Stoffgruppen können sogenannte Near-Infrared (NIR) Technologien eingesetzt werden, wobei über ein Spektrum von mehreren verschiedenen Wellenlängen simultan verschiedene Substanzen infrarot-optisch erfasst werden können.

Die erfassenden bzw. messenden Komponenten X sind bevorzugt im gastrischen Segment der trans-pylorischen Baugruppe TPG angeordnet. Die erforderlichen elektronischen und optischen Komponenten sind beispielsweise ringartig um die Schaftkomponente des gastrischen Schaftsegmentes 2 herum, im Binnenraum des der gastrischen Ballons 4 oder 5 angeordnet.

Um eine optimierte volumetrische Messung zu ermöglichen, kann die zugehörige Sensorik X auch innerhalb des Pylorus oder im Bereich des duodenalen Bulbus angeordnet werden, bzw. in entsprechender Weise in der Schaftkomponente 2 der Einheit verbaut sein.

Die zugehörige Elektronik verfügt bevorzugt über eine Energiequelle, die kabellos, auf induktive Weise in Intervallen von beispielsweise 24 oder 48 Stunden aufgeladen werden kann. Die Elektronik zeichnet die jeweiligen Parameter kontinuierlich auf. Der Anwender kann den Parameterverlauf beispielsweise mittels eines Mobiltelefons über eine entsprechende Bluetooth-Funktion oder eine sonstige drahtlose Funktion abrufen.

Die volumetrische Messung ermöglicht es dem Anwender seine Nahrungsaufnahme zu objektivieren und mit seinem, von ihm geführten Ernährungstagebuch zu vergleichen. Dem Anwender erschließt sich so ein Überblick über die tatsächliche Häufigkeit der Nahrungsaufnahme, wobei Nahrungsaufnahmen in kurzen Abständen in vielen Fällen ein wesentlicher kausaler Beitrag für die Entstehung von Übergewicht und einer diabetischen Stoffwechsellage sind.

In Folgenden wird der Ablauf der Applikation einer beispielhaften Ausführungsform der Vorrichtung beschrieben, wobei die Bypass-Einheit auf der gastrischen Seite ihrer trans-duodenalen Anordnung zwar über eine konzentrische, doppelte Ballonanordnung verfügt, aber nur der innenliegende Ballon befüllt wird. Der Schaftkörper der Positionierungs-Einheit ist hier durchgängig hergestellt, und wird bei der Entfaltung der Schlauchfolien-Komponente aus seiner Position innerhalb der trans-pylorischen Baugruppe entkoppelt und in das Duodenum vorgeschoben.
1. Die Applikation der Vorrichtung beginnt mit der endoskopisch geführten Anlage eines Führungsdrahtes. Das Endoskop wird hierzu nach Möglichkeit bis zum Treitzschen Band (Flexura duodenojejunalis) vorgeschoben. Von dort aus wird ein Führungsdraht so tief wie möglich in das sich dem Duodenum anschließenden Jejunum eingebracht. Alternativ zu einem Führungsdraht kann im Rahmen der Erfindung eine Kombination aus einem Draht und einem, die Biegeeigenschaften des Drahtes modifizierende Kathetereinheit über den Arbeitskanal des Endoskopes appliziert werden. Die Kathetereinheit kann mit einer distal angebrachten, von extrakorporal befüllbaren Ballonkomponente versehen sein, die den schienend wirkenden Verbund von Draht und Katheter im jeweiligen Darmabschnitt vor Dislokation sichert.
2. Nach der Platzierung des Drahtes wird das Endoskop aus dem Magen zurückgezogen. Im Anschluss an die Trennung des Endoskops vom führenden Draht wird das Endoskop erneut in den Magen eingeführt, um eventuelle Verschlingungen des Drahtes im Magen auszuschließen.
3. Vor der trans-ösophagealen Einführung der Vorrichtung in den Magen werden die beiden Einheiten der Vorrichtung, die Bypass-Einheit BE und die Positionierungs-Einheit PE, durch Befüllung der koppelnden Ballonkomponente der Kopplungs-Einheit KE miteinander in stabiler, verschiebesicherer Weise verbunden.
4. Anschließend wird das proximale Ende des Führungsdrahtes bzw. des Verbundes eines Drahtes mit einem, die Biegeeigenschaften des Drahtes modifizierenden Katheter durch die apikale Öffnung der henkelartigen Formation 8 geführt und durch das den Draht führende Lumen der Schaftkomponente 9 der Positionierungs-Einheit hindurch geschoben.
5. Nun erfolgt die eigentliche Einführung der Vorrichtung, indem der Verbund der beiden Einheiten PE und BE mit reichlich Gleitmittel bestrichen und dann über den führenden Draht bis auf eine Tiefe von etwa 70 cm eingeführt wird. Der Schaftkörper der Positionierungseinheit verfügt hierzu über entsprechende Tiefenmarkierungen. Die Referenz für die Einführtiefe stellt die vordere Zahnreihe des Patienten dar. Bei einer bevorzugten Gesamtlänge der Positionierungs-Einheit von 1,55 m, verbleiben etwa 85 cm der Schaftlänge vor der Zahnreihe, also außerhalb des Körpers. Die Position der Vorrichtung im Magen sollte nun durch ein Endoskop kontrolliert werden, das bis in den Bereich des unteren Korpus des Magens vorgeschoben wird.
6. Unter endoskopischer Kontrolle erfolgt nun der weitere Vorschub der Vorrichtung, bis der duodenale Ballon seine post-pylorische Position erreicht hat. Der gastrische Anteil der trans-pylorischen Baugruppe der Bypass-Einheit verbleibt im Magen.
7. Anschließend erfolgt die Befüllung des Ballons mit Flüssigkeit, beispielsweise etwa 10 ml NaCl-Lösung. Die Befüllung erfolgt unter endoskopischer Kontrolle.
8. Nachfolgend erfolgt die Befüllung des innenliegenden gastrischen Ballons 4 mit 2ml Methylenblau 0,5% an, die durch ca. 52 ml NaCl-Lösung ergänzt wird. Die Befüllung des inneren Ballons richtet nimmt den äußeren Ballon mit sich bzw. richtet diesen auf. Die korrekte trans-pylorische Position der Baugruppe wird wiederum endoskopisch dokumentiert.
9. In der Folge wird der koppelnde Ballon der Kopplungs-Einheit entleert, wodurch die Einheit PE gelöst und duodenalwärts vorgeschoben werden kann. Der Vorschub erfolgt bis zur Wahrnehmung eines spürbaren Widerstands. Das proximale Ende des Schaftkörpers der Positionierungs-Einheit ragt dann nur noch etwa 10 bis 20 cm über die Zahnreihe. Der Folienschlauch der trans-duodenalen Baugruppe ist nun vollständig axial entfaltet. Es erfolgt eine erneute endoskopische Lagekontrolle der trans-pylorischen Baugruppe.
10. Vor dem Rückzug der Einheit PE aus dem axial entfalteten Folienschlauch werden über eine kanalartige Zuleitung im Schaft der Einheit PE zunächst 50 ml Luft gefolgt von 100 ml NaCl-Lösung in den Binnenraum der Schlauchfolie eingebracht. Dann erfolgt der zeitgleiche vollständige Rückzug der Einheit PE sowie des Führungsdrahtes aus dem Patienten.
11. Anschließend wird das Endoskop in den Eingangsbereich der Baugruppe TPG eingeführt und über den Instrumentenkanal mit 50 ml NaCl-Lösung eine Spülung der Bypass-Einheit zur Prüfung der Durchgängigkeit durchgeführt.
12. Abschließend werden die Befüllschläuche der im Patienten verweilenden Bypass-Einheit mit einer Schlauchstanze oder einem anderen geeigneten endoskopischen Element einzeln durchtrennt und die Ansätze herausgezogen.

Die beschriebene Methode kann im Rahmen der vorliegenden Erfindung dahingehend modifiziert werden, dass die adhäsiven Wirkungen der Schlauchfolie auf die ihr anliegende Darmschleimhaut maximiert wird, indem
- vor der trans-ösophagealen Einführung der Vorrichtung eine wasser- oder fettbasierte Substanz auf den paketartig gerafften Folienschlauch aufgetragen wird, die die Adhäsion der Folie an der Schleimhaut verbessert;
- vor der Retraktion der Positionierungseinheit aus der axial entfalteten Schlauchfolie eine proximal und distal wirksame, lumenverschließende Dichtung des Binnenlumens der Schlauchfolie hergestellt wird, der anschließend eine Beaufschlagung des Lumens mit einem moderaten Fülldruck folgt, wobei sich die Schlauchfolie radial vollständig entfaltet und im Lumen des Darms den größtmöglichen adhäsiv wirkenden Kontaktbereich zur Schleimhaut erreicht.

Alternativ oder ergänzend hierzu können adhäsive und reibend wirkende Effekte zwischen der Wandung des Folienschlauches und der darin geführten Positionierungs-Einheit minimiert werden, indem
- in den Binnenraum des Folienschlauches Substanzen eingebracht werden, die lubrifizierend oder/oder anti-adhäsiv wirken;
- ein vorteilhaftes Verhältnis der jeweiligen Umfänge der Schlauchfolie und des Umfangs der darin geführten Schaftes oder Schaftanteils der Positionierungs-Einheit von 3,5 : 1 bis 7 : 1 nicht unterschritten wird;
- im Moment der Retraktion oder unmittelbar zuvor ein flüssiges oder gasförmiges Medium in den Binnenraum des Folienschlauches eingebracht wird, das zu einer passageren, partiellen Trennung der Kontaktflächen zwischen dem Schlauch und der Einheit PE führt.

Sind etwa 75% der Länge des Bypass-Schlauchkomponente entfaltet, kann der verbleibende, noch geraffte Anteil des folienartigen Schlauches durch eine Spülung des Schlauchlumens mit Wasser auf seine gesamte Länge entwickelt werden. Die Spülflüssigkeit tritt durch eine oder mehrere, ausreichend großlumige Öffnungen distal bzw. unterhalb des Kopplungsballons aus dem Schaftkörper der Positionierungs-Einheit bzw. ein darin integriertes, die Flüssigkeit zuleitendes Lumen, in den Binnenraum des Bypass-Schlauches aus und läuft in das tiefere Duodenum ab, wobei das abfließende Volumen den noch gerafften Bypass-Schlauch mit sich trägt und vollständig entfaltet.

Gelingt mit der spülenden Zuleitung von Flüssigkeit keine vollständige axiale Entfaltung des Schlauches, kann optional über den führenden Draht, durch das zentrale Lumen des Schaftes der Positionierungs-Einheit hindurch, eine zusätzliche Kathetereinheit bis an das distale Ende des Bypass-Schlauches geschoben werden. Die Kathetereinheit trägt am distalen Ende eine von extra-korporal befüllbare Ballonkomponente, die nach dem Austritt des Katheters aus der distalen Öffnung der Positionierungs-Vorrichtung mit Flüssigkeit befüllt und auf einen Durchmesser von ca. 10 bis 20 0m aufgedehnt wird. Beim Auftreffen des Ballons auf den henkelartigen Fortsatz des Bypass-Schlauches wird dieser bei weiterem Vorschub der Kathetereinheit schließlich vollständig in der Länge entwickelt.

**Bezugszeichenliste**

| | | | |
|---|---|---|---|
| 1 | System | 11e | Sphärische Erweiterung |
| 2 | Schaftkomponente | 11f | Zylindrischer Ballonkörper |
| 2a | Dichtungskomponente | 12 | Schlauchzuleitung |
| 2b | Dichtungskomponente | 13 | Lumen |
| 2c | Korrugation | 14 | Öffnung |
| 3 | Ballonkomponente | 15 | Katheter, Kathetereinheit |
| 3a | Zuleitender Schlauch | 16 | Ballon, Ballonkörper |
| 3b | Übergangsbereich | 17 | Formation |
| 4 | Ballonkomponente | 18 | Öffnung |
| 4a | Schlauch | 20 | Oberflächenwirksame Substanz |
| 4b | Übergangsbereich | 21 | Gelartige Substanz |
| 5 | Ballonkomponente | 22 | "Residuale" Faltung |
| 5a | Zuleitender Schlauch | | |
| 5b | Übergangsbereich | | |
| 6a | schlauchartige Zuleitung | | |
| 6b | schlauchartige Zuleitung | | |
| 7 | Schlauch, Schlauchfolie | | |
| 8 | Schlaufe | | |
| 9 | Schaftkomponente | | |
| 9a | Öffnung | | |
| 10 | Konnektor | | |
| 11 | Koppelnder Ballon | | |
| 11a | Erweiterung | | |
| 11b | Erweiterung | | |
| 11c | Wandung | | |
| 11d | Wandung | | |
| AT | Antrum-Bereich | | |
| BE | Bypass-Einheit | | |
| DS | Darmschleimhaut | | |
| DU | Duodenum | | |
| EN | Endoskop | | |
| ES | Oesophagus | | |
| GW | Führungsdraht | | |
| JE | Jejunum | | |
| K | Kanal | | |
| KE | Koppel-Einrichtung | | |
| OE | Öffnung | | |
| PE | Positionier-Einheit | | |
| PR | Profilgebung | | |
| PV | Positionier-Vorrichtung | | |
| PVS | Spitzenformation | | |
| PY | Pylorus | | |
| ST | Magen | | |
| TPG | Trans-pylorische Baugruppe | | |
| TDG | Trans-duodenale Baugruppe | | |
| X | Messkomponente, Sensorik | | |

## Patentansprüche

1. System (1) für eine bypass-artige, trans-duodenale Durchleitung von Mageninhalt in die höheren Anteile des Jejunums (JE) eines Patienten, umfassend
a) eine im Körper des Patienten verbleibende, trans-duodenale Bypass-Einheit (BE), umfassend (i) eine trans-duodenale Baugruppe (TDG) mit einem Folienschlauch (7) für die bypass-artige, trans-duodenale Durchleitung von Mageninhalt in die höheren Anteile des Jejunums (JE), sowie (ii) für eine Fixierung oder Verankerung der Bypass-Einheit (BE) im Bereich des Schließmuskels des Magenausgangs (Pylorus) eine trans-pylorische Baugruppe (TPG) mit einer schlauch- oder rohrartigen Schaftkomponente (2), und
b) für die Applikation der trans-duodenalen Bypass-Einheit (BE), also für deren trans-ösophageale Passage bzw. Einbringung, deren trans-pylorische Positionierung, sowie deren trans-duodenale Entfaltung einer die Bypass-Funktion gewährleistenden Schlauchfolie eine trans-ösophageale Positionier-Einheit (PE) mit einer vorzugsweise schlauchförmigen Schaftkomponente (9), deren Länge derart bemessen ist, dass sie von extrakorporal durch den Ösophagus, den Magen und wenigstens bis zum Treitzschen Band im Bereich des aboralen Endes des Duodenums reicht, und deren Außendurchmesser zumindest in einem distalen Bereich kleiner ist als der Innendurchmesser der Schaftkomponente (2) der trans-pylorischen Baugruppe (TPG) der Bypass-Einheit (BE),
**gekennzeichnet durch** eine von extra-korporal steuerbare Koppel-Einrichtung (KE), welche die trans-duodenale Bypass-Einheit (BE), insbesondere deren Schaftkomponente (2), auf der trans-ösophagealen Positionier-Einheit (PE), insbesondere auf deren Schaftkomponente (9), wahlweise koppelnd fixiert oder entkoppelnd freigibt.

2. System (1) nach Anspruch 1, **dadurch gekennzeichnet, dass** die Koppel-Einrichtung (KE) einen vorzugsweise hohlzylindrisch präformierten Ballon (11) aufweist, welcher innerhalb des Ringspaltes zwischen einem distalen Bereich der Schaftkomponente (9) der Positionier-Einheit (PE) einerseits und der Schaftkomponente (2) der trans-pylorischen Baugruppe (TPG) der Bypass-Einheit (BE) andererseits platzierbar und dort befüllbar ist, um die Bypass-Einheit (BE) auf der Positionier-Einheit (PE) dislokationssicher festzulegen.

3. System (1) nach einem der vorhergehenden Ansprüche, **dadurch gekennzeichnet, dass** ein hohlzylindrisch präformierter Ballon (11) der Koppel-Einrichtung (KE)
a) aus einem Material mit einer geringen Volumendehnbarkeit (Compliance) besteht, bspw. aus einem thermoplastischen Polyurethan der Härte nach Shore von 90A bis 99A oder von 55D bis 65D, und/oder
b) eine Wandungsstärke von wenigstens 10 µm aufweist, vorzugsweise eine Wandungsstärke von 15 µm oder mehr, und/oder eine Wandungsstärke von höchstens 40 µm aufweist, vorzugsweise eine Wandungsstärke von 30 µm oder weniger, und/oder
c) mit einem flüssigen Medium befüllbar ist, bspw. mit Wasser oder einer Flüssigkeit auf Wasserbasis, insbesondere über einen nur für diesen Zweck vorgesehenen Befüllungsschlauch oder -lumen.

4. System (1) nach einem der vorhergehenden Ansprüche, **dadurch gekennzeichnet, dass** ein hohlzylindrisch präformierter Ballon (11) der Koppel-Einrichtung (KE)
a) mit der Bypass-Einheit (BE) weder integriert noch verbunden ist, und/oder
b) mit der Positionier-Einheit (PE) integriert oder verbunden ist, insbesondere zumindest bereichsweise adhäsiv an dem distalen Bereich der Schaftkomponente (9) der Positionier-Einheit (PE) festgelegt ist, wobei die Oberfläche des Ballons (11) entweder schlauchförmig mit offenen Enden gestaltet ist und im Bereich der Schlauchenden an der Schaftkomponente (9) der Positionier-Einheit (PE) festgelegt sein kann, oder torusförmig geschlossen ist und im Bereich seiner Innenseite an der Schaftkomponente (9) der Positionier-Einheit (PE) festgelegt sein kann, und/oder
c) mit der Positionier-Einheit (PE) weder integriert noch verbunden ist und eine nach Art eines Torus geschlossene Hülle aufweist.

5. System (1) nach einem der vorhergehenden Ansprüche, **dadurch gekennzeichnet, dass** ein hohlzylindrisch präformierter Ballon (11) der Koppel-Einrichtung (KE)
a) im Bereich wenigstens einer Stirnseite eine vorzugsweise rundum laufende, radial nach außen vorspringende Erweiterung aufweist, und/oder
b) im Bereich beider Stirnseiten je eine vorzugsweise rundum laufende, radial nach außen vorspringende Erweiterung aufweist, vorzugsweise wobei der in Richtung der zentralen Symmetrieachse des hohlzylindrischen Ballons gemessene Abstand zwischen den beiden stirnseitigen Erweiterungen gleich oder größer ist als die Länge der Schaftkomponente (2) der Bypass-Einheit (BE).

6. System (1) nach einem der vorhergehenden Ansprüche, **gekennzeichnet durch** eine oder mehrere, an dem Schaftkomponente (2) der trans-pylorische Baugruppe (TPG) befestigten Ballonkomponenten (3,4,5) für die langfristige, atraumatisch organverträgliche Fixierung oder Verankerung der Bypass-Einheit (BE) im Bereich des Schließmuskels des Magenausgangs (Pylorus), wobei die befüllenden Zuleitungen zu den retinierend wirkenden duodenal- und magenseitig angeordneten Ballonkomponenten (3,4,5) über ringförmige, der radial außen liegenden Oberfläche der durchleitenden Schaftkomponente (2) der trans-pylorischen Baugruppe (TPG) der Bypass-Einheit (BE) unter elastischer Spannung anliegende Dichtungskomponenten (2b) angeschlossen sind.

7. System (1) nach einem der vorhergehenden Ansprüche, **dadurch gekennzeichnet, dass** ringförmige Dichtungskomponenten (2b)
a) aus einem Material mit dauerhaften, nicht ermüdenden elastischen Dehnungseigenschaften bestehen, bspw. aus Polyurethan oder Silikon, und/oder
b) an der (den) jeweilige(n), der betreffenden Ballonkomponente (3,4,5) zugeordneten Öffnung(en) in der Schaftkomponente (2) der Bypass-Einheit (BE) in dichtender Weise aufliegen, vorzugsweise die Ränder der betreffenden Öffnung(en) allseits flächig überlappend, und/oder
c) sich zwischen jeweils zwei Erhebungen befinden, welche den (die) ventiltragenden Abschnitt(e) der Schaftkomponente (2) der Bypass-Einheit (BE) an der jeweiligen Außenseite jeweils zirkulär umlaufen und einen gegenseitigen Abstand aufweisen, welcher der Breite der betreffenden, ringförmigen Dichtungskomponente (2b) entspricht, und die ringförmigen Dichtungskomponenten (2b) in einbettender Weise zwischen sich sichernd aufnehmen.

8. System (1) nach einem der vorhergehenden Anspruche, **dadurch gekennzeichnet, dass** befüllende Zuleitungen (3a,4a,5a) zu den retinierend wirkenden duodenal- und/oder magenseitig angeordneten Ballonkomponenten (3,4,5)
a) an der Innenseite der Schaftkomponente (2) der Bypass-Einheit (BE) angeschlossen sind, und zwar mit jeweils einer Öffnung in der Schaftkomponente (2) der Bypass-Einheit (BE) kommunizierend, und/oder
b) in distalen Bereichen, vorzugsweise nahe der Schaftkomponente (2), zumindest bereichsweise mit einer reduzierten Wandstärke präformiert sind, um von einem scheren- oder zangenartigen Werkzeug durchtrennt werden zu können.

9. System (1) nach einem der vorhergehenden Ansprüche, **dadurch gekennzeichnet, dass** die Bypass-Einheit (BE)
a) eine distale Ballonkomponente für die Positionierung im Bulbus duodeni aufweist, bevorzugt aus TPU, die vollständig ausgeformt sein kann, und in schlaff befülltem Zustand auf ca. 80 bis 90 % ihres vorgeformten bzw. frei entfalteten Volumens aufgebläht wird, und bevorzugt mit einem Gas befüllt wird, bspw. mit Luft, und/oder
b) eine proximale Ballonanordnung für die Positionierung im Magen aufweist, mit zwei Ballons, die vorzugsweise ineinender eingehaust sind.

10. System (1) nach Anspruch 9, **dadurch gekennzeichnet, dass**
a) der innere proximale Ballon aus einer einzigen Lage besteht, insbesondere aus einem thermoplastischen Polyurethan (TPU), und/oder
b) der distale und der äußere proximale Ballon jeweils aus einem zweilagigen Material bestehen, das bevorzugt säurebeständig ist, und/oder
c) der innere proximale Ballon bevorzugt mit einer Flüssigkeit befüllt wird, und/oder
d) der äußere proximale Ballon bevorzugt mit einem Gas befüllt wird, bspw. mit Luft, oder unbefüllt bleibt.

11. System (1) nach einem der vorhergehenden Ansprüche, **dadurch gekennzeichnet, dass** die Schaftkomponente (2) der Bypass-Einheit (BE) eine Schaftinnenstruktur aus einem elastischen Polymer aufweist, bevorzugt blasgeformt oder spritzgegossen, vorzugsweise mit eingeformten Korrugationen und/oder mit integrierten Öffnungen und/oder Lumina für die Befüllung von Ballonkomponenten (3,4,5).

12. System (1) nach einem der vorhergehenden Ansprüche, **dadurch gekennzeichnet, dass** die Schlauchfolie (7) der trans-duodenalen Baugruppe (TDG) der Bypass-Einheit (BE), vorzugsweise im Bereich ihres distalen Endes, eine Schlinge oder eine henkel- oder schleifenartige Formation aufweist, vorzugsweise wobei die jeweilige Schleifen- oder Henkelformation eine apikale Stanzung für die Durchführung einer Führungsdraht-Komponente oder finger-artigen Aufnahmekomponente der Spitze der Positionier-Einheit (PE) aufweist.

13. System (1) nach einem der vorhergehenden Ansprüche, **dadurch gekennzeichnet, dass** die Schlauchfolie (7) der trans-duodenalen Baugruppe (TDG) der Bypass-Einheit (BE) im nicht entfalteten Zustand gerafft ist, vorzugsweise auf etwa 10 cm.

14. System (1) nach einem der vorhergehenden Ansprüche, **dadurch gekennzeichnet, dass** die Summe der adhäsiven und/oder reibenden Wirkungen der äußeren Wandung des Folienschlauches zur ihr exponierten Darmschleimhaut, die Summe der adhäsiven und reibenden Wirkungen zwischen der inneren Wandung des Folienschlauches und des in ihr bewegten Schaftanteils der Positionier-Einheit (PE) überschreitet, wodurch bei der Retraktion des distalen Anteils der Positionier-Einheit (PE) aus dem duodenal und jejunal entfalteten Folienschlauch der Bypass-Einheit (BE) eine nach oral gerichtete Raffung des Folienschlauches vermieden wird.

15. System (1) nach einem der vorhergehenden Ansprüche, **dadurch gekennzeichnet, dass** die adhäsiven und/oder reibenden Wirkungen zwischen der inneren Wandung der Schlauchfolie (7) und des in ihr bewegten Schaftanteils der Positionier-Einheit (PE) reduziert werden durch
a) eine Einrichtung zur Einbringung von reibungs- und/oder adhäsionsreduzierenden Substanzen in den Binnenraum des Folienschlauches, und/oder
b) durch eine Einrichtung zur Insufflation von Wasser oder Luft in den Binnenraum des Folienschlauches, welche die adhäsiven und reibenden Wirkungen zwischen der inneren Wandung des Folienschlauches und des in ihr bewegten Schaftanteils der Positionier-Einheit (PE) reduziert.

16. System (1) nach einem der vorhergehenden Ansprüche, **dadurch gekennzeichnet, dass** die Summe der adhäsiven und/oder reibenden Wirkungen der äußeren Wandung der Schlauchfolie (7) zur ihr exponierten Darmschleimhaut dadurch erhöht werden, dass auf der äußeren Oberfläche der Schlauchfolie (7) und/oder zwischen der der äußeren Oberfläche der Schlauchfolie (7) und der Darmschleimhaut (DS) ein oder mehrere reibungs- und/oder adhäsionserhöhende Substanzen, bspw. eine oder mehrere Pasten, Gele oder gelartig quellende Substanzen (21), aufgetragen oder eingebracht sind.

17. System (1) nach einem der vorhergehenden Ansprüche, **dadurch gekennzeichnet, dass** die adhäsiven und reibenden Wirkungen der äußeren Wandung der Schlauchfolie (7) zur ihr exponierten Darmschleimhaut erhöht werden mittels
a) einer Einrichtung zur passagerne Beaufschlagung des dortigen, allseits abgeschlossenen Binnenraumes innerhalb der Schlauchfolie (7) mit einem moderaten Druck, wodurch sich im Zustand der vollständigen Entfaltung der Schlauchfolie (7) innerhalb des Darmlumens die äußere Oberfläche der Schlauchfolie (7) vollständig flächig an die Schleimhaut anlegt und somit ein größtmöglicher Adhäsionseffekt hergestellt wird, und/oder
b) mittels einer Einrichtung zum passageren Verschluss des proximalen und des distalen Endes des Folienschlauches, damit der solchermaßen abgeschlossene Binnenraum innerhalb des Folienschlauchs mit einem moderaten Druck beaufschlagt werden kann.

18. System (1) nach einem der vorhergehenden Ansprüche, **dadurch gekennzeichnet, dass** das Verhältnis des Durchmessers der äußeren Umfangs- oder Querschnittsfläche der Schaftkomponente (9) der Positionier-Einheit (PE) zum Durchmesser der Schlauchfolie (7) der trans-duodenalen Baugruppe (TDG) der Bypass-Einheit (BE) von 1 : 2 oder weniger, bevorzugt durch ein Verhältnis von 1 : 3 oder weniger, insbesondere durch ein Verhlältnis von 1 : 4 oder weniger, damit die adhäsiven und/oder reibenden Wirkungen zwischen der inneren Wandung der Schlauchfolie (7) und des in ihr bewegten Berreichs der Schaftkomponente (9) der Positionierungs-Einheit (PE) reduziert werden.

19. System (1) nach einem der vorhergehenden Ansprüche, **dadurch gekennzeichnet, dass** die Schaftkomponente (9) der Positionier-Einheit (PE) aus einem einzigen Element mit durchgehendem Profil besteht, oder aus mehreren Segmenten mit unterschiedlichen Profilen zusammengesetzt ist, vorzugsweise wobei
a) ein Segment am distalen Ende der Schaftkomponente (9) der Positionier-Einheit (PE) eine höhere Biegsamkeit bzw. Flexibilität oder Elastizität aufweist als ein oder mehrere, proximal dazu angeordnete Segmente, und/oder wobei
b) zwei Segmente der Schaftkomponente (9) der Positionier-Einheit (PE) mit unterschiedlichen Profilen teleskopartig ineinander angeordnet sind und relativ zueinander verschiebbar sind, und/oder wobei
c) eine distale und/oder innere Schaftkomponente (9) der Positionier-Einheit (PE) einen distalen Ballon (16) trägt, welcher ankernd wirken kann, und/oder das distale Lumen der Folienschlauch-Komponente verschließen kann, und/oder eine henkelartige Struktur (18) am distalen Ende der Schlauchfolie (7) nach aboral führen kann.

20. System (1) nach einem der vorhergehenden Ansprüche, **dadurch gekennzeichnet, dass** die Positionier-Einheit (PE) ein inneres Lumen aufweist, worin ein vorzugsweise endoskopisch verlegter Führungsdraht (GW), ggf. zusammen mit einem jenen Führungsdraht (GW) umschließenden, kleinlumigen Ballonkatheter (15) geführt werden kann, vorzugsweise wobei der Führungsdraht (GW) zumindest bereichsweise von einem kleinlumigen Ballonkatheter (15) umschlossen ist, der über den Führungsdraht (GW) oder gemeinsam mit dem Führungsdraht (GW) einbringbar ist, und der im befüllten Zustand ankernd wirkt und zusätzlich zur Fixierung des distalen Schlauchfolie (7) verwendet werden kann, während die Positionier-Einheit (PE) zurückgezogen wird.
